# EUROPEAN PATENT SPECIFICATION

(11) **EP 3 730 054 B1**
(45) Date of publication and mention of the grant of the patent: **27.03.2024**
(21) Application number: 18891882.5
(22) Date of filing: 30.11.2018
(51) Int. Cl.: A61B 5/18, A61B 5/026, A61B 5/0245, A61B 5/1455, A61B 5/00

(54) **BIOLOGICAL MEASUREMENT APPARATUS**
BIOLOGISCHE MESSVORRICHTUNG
APPAREIL DE MESURE BIOLOGIQUE

(30) Priority: 19.12.2017 JP 2017243045
(43) Date of publication of application: 28.10.2020
(73) Proprietor: Panasonic Intellectual Property Management Co., Ltd., Osaka-shi, Osaka 540-6207 (JP)
(72) Inventor: ANDO, Takamasa, Osaka-shi, Osaka 540-6207 (JP); SUZUKI, Masato, Osaka-shi, Osaka 540-6207 (JP)
(74) Representative: Grünecker Patent- und Rechtsanwälte PartG mbB
(86) International application number: PCT/JP2018/044173
(87) International publication number: WO 2019/124023

(56) References cited:
- WO-A1-2015/175435
- WO-A1-2017/090487
- WO-A1-2017/108766
- JP-A- 2003 339 648
- JP-A- 2005 253 590
- JP-A- 2011 010 714
- JP-A- 2011 010 714
- JP-A- 2015 134 157
- JP-A- 2017 009 584
- JP-A- 2017 011 693
- US-A1- 2012 212 353
- US-A1- 2017 225 677
- BOVEROUX PIERRE ET AL: "Brain Function in Physiologically, Pharmacologically, and Pathologically Altered States of Consciousness", INTERNATIONAL ANESTHESIOLOGY CLINICS., [Online] vol. 46, no. 3, 1 January 2008 (2008-01-01), pages 131-146, XP093078590, US ISSN: 0020-5907, DOI: 10.1097/AIA.0b013e318181a8b3 Retrieved from the Internet: URL:http://dx.doi.org/10.1097/AIA.0b013e31 8181a8b3> [retrieved on 2023-09-05]

## Description

The present disclosure relates to a biometric apparatus and a biometric method,

In the related art, a method of determining the state of a target person from the movements of the target person or an image obtained by taking a picture of the target person is known. For example, Patent Literature 1 discloses a method of determining whether or not a driver is drowsy from steering wheel operations by the driver. Also, Patent Literature 2 discloses a method of determining an open eye state from an image obtained by taking a picture of a driver with a camera, and determining whether or not the driver is drowsy.

PTL 1: Japanese Unexamined Patent Application Publication No. 2004-310738
PTL 2: Japanese Unexamined Patent Application Publication No. 2017-143889

US 2017/225677 A1 discloses a measuring instrument that measures the level of uneasiness of a driver during autonomous control of a vehicle. The measurement instrument uses brain wave sensors to measure the brain activity. Uneasiness of the driver is detected in case the measured brain activity is larger than a reference value.

JP 2011 010714 A describes a wearable biological signal detection device. The detection device monitors brain activity areas in addition to the heart rate of a subject. For this purpose, the detection device irradiates the scalp of the subject and detects the reflected light.

WO 2015/175435 A1 describes a monitoring system in a vehicle that determines if the driver becomes incapable of controlling the vehicle. The monitoring system uses capacitance sensors mounted on the seatbelt to detect brain waves and a camera to detect biological signals. The monitoring system determines from the captured data whether the driver is drowsy, falling asleep or incapable of controlling the vehicle.

US 2012/212353 A1 discloses a system that monitors the drowsiness of a driver. The drowsiness is detected based on information acquired by different sensors located throughout the vehicle. The various sensors measure the heart rate, respiration and brain information.

WO 2017/108766 A1 relates to a method of determining sleep stages based on EEG data. A sensor attached to the skin of a subject acts as an EEG electrode detecting electrical signals.

JP 2005-253590 A describes the determination of the consciousness state of the driver of a vehicle and respective actions to be taken by the vehicle depending on the determined consciousness state of the driver. There are four different states determined based on two measured signals, namely the measured bloodstream of the driver's brain and the measured driver's "recognition". The bloodstream is determined based on scattered light of near infrared light irradiated to the driver's head by a light emitter and sensor mounted to the driver's head. The measurement of the second signal is performed by measuring the recognition of the driver is measured by a camera and an analysis of the gaze of the driver in an imageprocessing part. A state of reduced consciousness is determined when the change in the blood flow is reduced.

The technologies of the related art require movements by the target person to detect the state of the target person, and the determinable states are limited.

The present disclosure provides a novel technology capable of determining various states of a target person without requiring movements by the target person.

This is achieved by the features of claim 1, which defines the apparatus of the present invention.

It should be noted that general or specific aspects may be implemented as an apparatus, a system, a method, an integrated circuit, a computer program, a storage medium, or any selective combination thereof.

According to the technology of the present disclosure, by using brain activity information and biological information other than brain activity information, it is possible to determine various states of a target person.

### Brief Description of Drawings

[Fig. 1A] Fig. 1A is a schematic diagram illustrating a biometric apparatus according to an exemplary embodiment of the present disclosure.
[Fig. 1B] Fig. 1B is a graph illustrating an example of change over time in the intensity of light reaching an image sensor.
[Fig. 1C] Fig. 1C is a graph illustrating the width of an input light pulse on the horizontal axis and the amount of light detected by a sensor on the vertical axis.
[Fig. 1D] Fig. 1D is a diagram illustrating an example of a schematic configuration of a pixel in the image sensor.
[Fig. 1E] Fig. 1E is a diagram illustrating an example of the configuration of the image sensor.
[Fig. 1F] Fig. 1F is a diagram illustrating an example of operations in a single frame.
[Fig. 1G] Fig. 1G is a flowchart illustrating an overview of operations by a control circuit.
[Fig. 2] Fig. 2 is a diagram for explaining a method of detecting an internal scattering component of a light pulse.
[Fig. 3A] Fig. 3A is a diagram illustrating an example of a timing chart in a case of detecting a surface reflection component.
[Fig. 3B] Fig. 3B is a diagram illustrating an example of a timing charge in a case of detecting an internal scattering component.
[Fig. 4A] Fig. 4A is a diagram schematically illustrating the relationship between a person's pulse, brain activity, and bodily state.
[Fig. 4B] Fig. 4B is a diagram schematically illustrating the relationship between a person's perspiration, brain activity, and bodily state.
[Fig. 4C] Fig. 4C is a diagram schematically illustrating the relationship between a person's body temperature, brain activity, and bodily state.
[Fig. 4D] Fig. 4D is a diagram schematically illustrating the relationship between a person's respiration, brain activity, and bodily state.
[Fig. 5] Fig. 5 is a diagram illustrating how the vital signs of a passenger in a self-driving taxi are detected by the biometric apparatus.
[Fig. 6] Fig. 6 is a diagram illustrating another exemplary configuration of a biometric apparatus.
[Fig. 7] Fig. 7 is a flowchart illustrating an example of operations by the biometric apparatus.
[Fig. 8] Fig. 8 is a diagram illustrating an example of another application of the biometric apparatus.
[Fig. 9] Fig. 9 is a diagram illustrating an example of yet another application of the biometric apparatus.
[Fig. 10] Fig. 10 is a diagram illustrating an example of yet another application of the biometric apparatus.
[Fig. 11A] Fig. 11A is a diagram illustrating a biometric apparatus integrated into a head-mounted display, which is an example of yet another application of the biometric apparatus.
[Fig. 11B] Fig. 11B is a diagram illustrating how a target person uses the biometric apparatus illustrated in Fig. 11A.
[Fig. 12] Fig. 12 is a diagram illustrating an example of yet another application of the biometric apparatus.
[Fig. 13] Fig. 13 is a diagram illustrating an example of yet another application of the biometric apparatus.
[Fig. 14] Fig. 14 is a diagram illustrating an example of yet another application of the biometric apparatus.
[Fig. 15] Fig. 15 is a diagram illustrating yet another exemplary configuration of the biometric apparatus.

### Description of Embodiments

The present disclosure includes a biometric apparatus, a biometric method, and a determination apparatus according to the following items.

### [Item 1]

A biometric apparatus according to Item 1 of the present disclosure is provided with a first detector that detects and outputs a brain activity signal indicating a state of brain activity in a target person, and a signal processing circuit.

The signal processing circuit acquires the brain activity signal, acquires a biological signal of the target person that is different from the brain activity signal, and based on the biological signal and the brain activity signal, determines whether the state of the target person is an awake state, a sleeping state, or a state of disturbed consciousness, and generates and outputs a signal indicating the state of the target person.

According to the above aspect, by using both the biological signal and the brain activity signal, the state of the target person can be determined more accurately than the related art.

### [Item 2]

In the biometric apparatus according to Item 1, the first detector may further detect and output the biological signal.

### [Item 3]

The biometric apparatus according to Item 1 may be further provided with a second detector that detects and outputs the biological signal.

### [Item 4]

The biometric apparatus according to any of Items 1 to 3 may be further provided with a light source that emits a light pulse irradiating a head of the target person, and a control circuit. The first detector may detect at least a part of a reflected light pulse returning from the head. The control circuit may cause the light source to emit the light pulse, and cause the first detector to detect and output an internal scattering component scattered inside a brain of the target person from the reflected light pulse as the brain activity signal.

### [Item 5]

In the biometric apparatus according to any of Items 1 to 4, the biological signal may include at least one type of information selected from the group consisting of pulse, perspiration, respiration, and body temperature.

According to the above aspects, it is possible to determine the state of the target person based on at least one piece of biological information selected from the group consisting of pulse, perspiration, respiration, and body temperature, and also determine the state of the target person based on the brain activity signal.

### [Item 6]

In the biometric apparatus according to any of Items 1 to 5, the brain activity signal may include information on a change in a quantity of cerebral blood flow in the target person.

According to the above aspect, it is possible to estimate the brain activity state of the target person based on information related to a change such as an increase or decrease in the quantity of cerebral blood flow in the target person.

### [Item 7]

In the biometric apparatus according to any of Items 1 to 6, the signal processing circuit may acquire the brain activity signal after acquiring the biological signal.

According to the above aspect, it is possible to first determine the state of the target person based on a biological signal other than a brain activity signal, and thereafter determine the brain activity state based on the brain activity signal.

### [Item 8]

In the biometric apparatus according to any of Items 1 to 7, the signal processing circuit may determine and output whether a pulse of the target person is bradycardia or tachycardia, based on the biological signal and the brain activity signal.

According to the above aspect, it is possible to determine if the pulse of the target person is bradycardia or tachycardia.

### [Item 9]

In the biometric apparatus according to any of Items 1 to 8, the biological signal may include information on a blood flow at a surface of a skin of the target person.

According to the above aspect, it is possible to determine the state of the target person based on information about blood flow at the surface of the skin of the target person.

### [Item 10]

In the biometric apparatus according to Item 4, the control circuit may cause the first detector to further detect a surface reflection component reflected at a surface of a skin of the target person from the reflected light pulse, and output a signal indicating a variation in the surface reflection component as the biological signal.

According to the above aspect, it is possible to acquire both information on the blood flow at the surface of the skin of the target person and information on cerebral blood flow with a single detector.

### [Item 11]

In the biometric apparatus according to Item 10, the control circuit may cause the first detector to detect the internal scattering component by detecting a portion of the reflected light pulse after an intensity of the reflected light pulse starts to decrease, and detect the surface reflection component by detecting a portion of the reflected light pulse before the intensity of the reflected light pulse starts to decrease.

According to the above aspect, it is possible to detect the internal scattering component and the surface reflection component of a light pulse returning from the head of the target person with a high SN ratio.

### [Item 12]

In the biometric apparatus according to Item 10 or 11, the first detector may be an image sensor including a plurality of pixels arranged in a two-dimensional matrix. Each of the plurality of pixels may include a photoelectric converter that converts received light into a signal charge, a first charge accumulator that accumulates the signal charge corresponding to the surface reflection component, and a second charge accumulator that accumulates the signal charge corresponding to the internal scattering component.

According to the above aspect, it is possible to acquire a two-dimensional image of the target person.

### [Item 13]

In the biometric apparatus according to Item 4, a time from a start of irradiation with the light pulse until an acquisition of the brain activity signal may be longer than a time from the start of irradiation with the light pulse until an acquisition of the biological signal.

According to the above aspect, it is possible to first make a diagnosis based on a biological signal other than a brain activity signal, and thereafter make a diagnosis based on the brain activity signal.

### [Item 14]

In the biometric apparatus according to any of Items 1 to 13, the signal processing circuit may make a first determination related to the state of the target person based on the biological signal, and outputs a first signal indicating a result of the first determination, and after outputting the first signal, make a second determination related to the state of the target person based on the brain activity signal, and outputs a second signal indicating a result of the second determination.

According to the above aspect, it is possible to first make a diagnosis based on a biological signal other than a brain activity signal, and thereafter make a diagnosis based on the brain activity signal.

### [Item 15]

The biometric apparatus according to Item 14 may be provided with a communication circuit that communicates with an external apparatus external to the biometric apparatus. When the signal processing circuit determines that the target person is not in a normal state based on at least one selected from the group consisting of the biological signal and the brain activity signal, the communication circuit may notify the external apparatus that the target person is not in the normal state.

According to the above aspect, in the case of determining that the target person is not in a normal state based on the biological signal and/or the brain activity signal, it is possible to notify an external apparatus in a medical institution for example. For this reason, even if the target person is in a dangerous state, it is possible to take prompt action such as dispatching an ambulance, for example.

### [Item 16]

A biometric method according to Item 17 of the present disclosure includes a step of acquiring a brain activity signal of a target person, a step of acquiring a biological signal of the target person that is different from the brain activity signal, and a step of determining, based on the biological signal and the brain activity signal, whether a state of the target person is an awake state, a sleeping state, or a state of disturbed consciousness.

According to the above aspect, by using both the biological signal and the brain activity signal, the state of the target person can be determined more accurately than the related art.

### [Item 17]

In the biometric method according to Item 16, the step of acquiring the brain activity signal may include a step of irradiating a head of the target person with a light pulse using a light source in a biometric apparatus that includes the light source, a photodetector, a signal processing circuit, and a control circuit, and a step of using the photodetector to detect and output an internal scattering component scattered inside a brain of the target person from a reflected light pulse returning from the head as the brain activity signal. The determining step may be performed using the signal processing circuit.

### [Item 18]

In the biometric method according to Item 17, based on a result of determining the state of the target person, the control circuit may perform any of controlling an output of a notification to the target person, controlling equipment near the target person that is different from the biometric apparatus, and communicating with an external apparatus that is external to the biometric apparatus.

### [Item 19]

In the biometric method according to any of Items 16 to 18, the biological signal may include at least one type of information selected from the group consisting of pulse, perspiration, respiration, and body temperature.

According to the above aspects, it is possible to determine the state of the target person based on at least one piece of biological information selected from the group consisting of pulse, perspiration, respiration, and body temperature, and also determine the state of the target person based on the brain activity signal.

### [Item 20]

In the biometric method according to any of Items 16 to 19, the brain activity signal may include information on a change in a quantity of cerebral blood flow in the target person.

According to the above aspect, it is possible to estimate the brain activity state of the target person based on information related to a change such as an increase or decrease in the quantity of cerebral blood flow in the target person.

### [Item 21]

In the biometric method according to any of Items 16 to 20, the step of acquiring the brain activity signal may be performed after the step of acquiring the biological signal.

According to the above aspect, it is possible to first determine the state of the target person based on on a biological signal other than a brain activity signal, and thereafter determine the brain activity state based on the brain activity signal.

### [Item 22]

In the biometric method according to any of Items 16 to 21, the determining step may include determining whether a pulse of the target person is bradycardia or tachycardia.

According to the above aspect, it is possible to determine if the pulse of the target person is bradycardia or tachycardia.

### [Item 23]

In the biometric method according to any of Items 16 to 22, the biological signal may include information on a blood flow at a surface of a skin of the target person.

According to the above aspect, it is possible to determine the state of the target person based on information about blood flow at the surface of the skin of the target person.

### [Item 24]

In the biometric method according to Item 17 or 18, the biometric apparatus may further include memory that stores a first standard value and a second standard value, the first standard value being a standard value of the biological signal, and the second standard value being a standard value of the brain activity signal, and the determining step may be performed by comparing the biological signal to the first standard value and comparing the brain activity signal to the second standard value.

According to the above aspect, the state of the target person based on the biological signal and the brain activity signal can be determined through a comparison of two different standard values.

### [Item 25]

In the biometric method according to Item 17 or 18, the biometric apparatus may further include memory that stores a data table expressing a relationship between the brain activity signal, the biological signal, and the state of the target person, and the determining step may be performed by referencing the data table.

According to the above aspect, by referencing a data table expressing the relationship between the brain activity signal, the biological signal, and the bodily state to make an evaluation, the bodily state of the target person may be determined more accurately.

### [Item 26]

The biometric method according to Item 17 or 18 may further include a step of acquiring time-series data of the biological signal and the brain activity signal. The biometric apparatus may further include memory that stores reference time-series data corresponding to the time-series data, and the determining step may include at least one selected from the group consisting of comparing the time-series data to the reference time-series data, computing a statistical value of the time-series data, and executing machine learning of a correlation between the time-series data and the state of the target person.

According to the above aspect, the state of the target person may be determined more accurately.

### [Item 27]

The biometric method according to Item 17 or 18 may further include a step of acquiring two-dimensional image data corresponding to the biological signal and the brain activity signal, and a step of extracting a feature value contained in the two-dimensional image data. The biometric apparatus may further include memory that stores a reference feature value corresponding to the feature value, and the determining step may include at least one selected from the group consisting of comparing the feature value to the reference feature value, computing a statistical value of the feature value, and executing machine learning of a correlation between the feature value and the state of the target person.

### [Item 28]

A determination apparatus according to Item 28 of the present disclosure is provided with one or more memories, and a circuit that, in operation, acquires a brain activity signal of a target person, acquires a biological signal of the target person that is different from the brain activity signal, and determines, based on the biological signal and the brain activity signal, whether a state of the target person is an awake state, a sleeping state, or a state of disturbed consciousness.

### [Item 29]

A biometric apparatus according to Item 29 of the present disclosure is provided with a light source that emits a light pulse irradiating a head of a target person, a photodetector that detects at least a part of a reflected light pulse returning from the head, a control circuit that controls the light source and the photodetector, and a signal processing circuit.

The control circuit causes the light source to emit the light pulse, causes the photodetector to detect and output the internal scattering component scattered inside the brain of the target person from the reflected light pulse as a brain activity signal, and causes the photodetector to detect and output the surface reflection component reflecting off the surface of the skin of the target person from the reflected light pulse as a biological signal different from the brain activity signal.

The signal processing circuit generates and outputs a signal indicating the state of the target person based on the biological signal and the brain activity signal.

### [Item 30]

In the biometric apparatus according to Item 29, the control circuit may cause the photodetector to detect the internal scattering component by detecting a portion of the reflected light pulse after an intensity of the reflected light pulse starts to decrease, and detect the surface reflection component at least by detecting a portion of the reflected light pulse before the intensity of the reflected light pulse starts to decrease.

### [Item 31]

In the biometric apparatus according to Item 29, the biological signal may include at least one type of information selected from the group consisting of pulse, perspiration, respiration, and body temperature.

The embodiments described hereinafter all illustrate general or specific examples. Features such as numerical values, shapes, materials, structural elements, and layout positions of structural elements indicated in the following exemplary embodiments are merely examples, and are not intended to limit the technology of the present disclosure. In addition, among the structural elements in the following embodiments, structural elements that are not described in the independent claim indicating the broadest concept are described as arbitrary or optional structural elements.

In the present disclosure, all or a part of any of circuit, unit, device, part or portion, or any of functional blocks in the block diagrams may be implemented as one or more of electronic circuits including, but not limited to, a semiconductor device, a semiconductor integrated circuit (IC) or a large scale integration (LSI). The LSI or IC can be integrated into one chip, or also can be a combination of plural chips. For example, functional blocks other than a memory may be integrated into one chip. The name used here is LSI or IC, but it may also be called system LSI, very large scale integration (VLSI), or ultra large scale integration (ULSI) depending on the degree of integration. A field programmable gate array (FPGA) that can be programmed after manufacturing an LSI or a reconfigurable logic device that allows reconfiguration of the connection or setup of circuit cells inside the LSI can be used for the same purpose.

Further, it is also possible that all or a part of the functions or operations of the circuit, unit, device, part or portion are implemented by executing software. In such a case, the software is recorded on one or more non-transitory recording media such as ROM, an optical disk or a hard disk drive, and when the software is executed by a processor, the software causes the processor together with peripheral devices to execute the functions specified in the software. A system or apparatus may include such one or more non-transitory recording media on which the software is recorded and a processor together with necessary hardware devices such as an interface.

Hereinafter, embodiments will be described specifically with reference to the drawings. In the following description, identical or similar components are denoted by the same signs.

### (Embodiments)

### [1. Biometric apparatus]

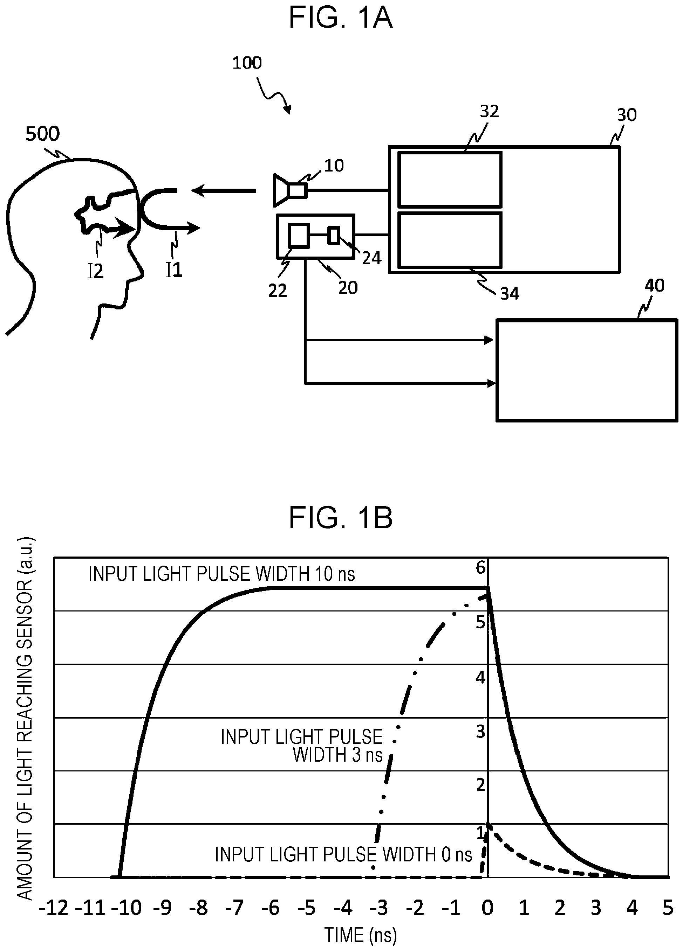

Figs. 1A to 3B will be referenced to describe a configuration of a biometric apparatus 100 according to an exemplary embodiment of the present disclosure.

Fig. 1A is a schematic diagram illustrating the biometric apparatus 100 according to the present embodiment. The biometric apparatus 100 is provided with a light source 10, an image sensor 20, a control circuit 30, and a signal processing circuit 40. The image sensor 20 includes a plurality of photoelectric converters 22 and a plurality of charge accumulators 24. The image sensor 20 is an example the first detector, the second detector, and the photodetector in the present disclosure. Instead of the image sensor 20, it is also possible to use another type of photodetector including at least one photoelectric converter 22 and at least one charge accumulator 24.

The light source 10 emits a light pulse irradiating the head of a target person 500. The image sensor 20 detects at least a part of a reflected light pulse, which is a light pulse returning from the head of the target person 500. The control circuit 30 controls the light source 10 and the image sensor 20. The signal processing circuit 40 processes the signal output from the image sensor 20.

In the present embodiment, the control circuit 30 includes a light source controller 32 that controls the light source 10 and a sensor controller 34 that controls the image sensor 20. The light source controller 32 controls the intensity, pulse width, emission timing, and/or wavelength of the light pulse emitted from the light source 10. The sensor controller 34 controls the timing of signal storage in each pixel of the image sensor 20.

Hereinafter, each of these components will be described in further detail.

### [1-1. Light source 10]

The light source 10 irradiates the head (for example, the forehead) of the target person 500 with light. The light emitted from the light source 10 and reaching the target person 500 is divided into a surface reflection component I1 that is reflected at the surface of the target person 500 and an internal scattering component I2 that is scattered inside the target person 500. The internal scattering component I2 is the component that reflected or scattered once or rescattered inside a living body. In the case of irradiating the forehead of the target person 500 with light, the internal scattering component I2 refers to the component that reaches a site 8 mm to 16 mm deep from the surface of the forehead, such as the brain for example, and returns again to the biometric apparatus 100. The surface reflection component I1 includes three components: a direct reflection component, a diffuse reflection component, and a scattered reflection component. The direct reflection component is the reflection component for which the angle of incidence is equal to the angle of reflection. The diffuse reflection component is the component that is diffusely reflected by the uneven shape of the surface. The scattered reflection component is the component whose reflection is scattered by internal tissue near the surface. In the case of irradiating the forehead of the target person 500 with light, the scattered reflection component is the component whose reflection is scattered inside the epidermis. Hereinafter, the present disclosure assumes that the surface reflection component I1 reflected at the surface of the target person 500 includes these three components. The direction of travel by the surface reflection component I1 and the internal scattering component I2 varies depending on the reflection or scattering, and a part of each of these components reaches the image sensor 20.

First, a method of acquiring the internal scattering component I2 will be described. The light source 10, following an instruction from the control circuit 30, repeatedly produces light pulses at predetermined time intervals or predetermined timings. Each light pulse produced by the light source 10 may be a square wave whose falling time is near-zero, for example. The falling time refers to the time from when the intensity of the light pulse starts to fall from a peak value until reaching near-zero. Typically, the light incident on the target person 500 propagates inside the target person 500 along various paths, and is emitted from the surface of the target person 500 after a time difference. For this reason, the trailing edge of the internal scattering component I2 of the light pulse spreads out. In the case where the target area is the forehead, the trailing edge of the internal scattering component I2 spread over approximately 4 ns. Taking the above into consideration, the falling time of the light pulse may be set to half of the spread or less, such as 2 ns or less for example. The falling time may also be halved again to 1 ns or less. The light pulse produced by the light source 10 may have any rising time. This is because the detection of the internal scattering component I2 in the present embodiment uses the falling portion of the light pulse and does not use the rising portion. The rising portion of the light pulse may be used to detect the surface reflection component I1. For example, the light source 10 may be a laser such as an LD in which the falling portion of the light pulse is nearly a right angle with respect to the time axis, or in other words, a laser having fast time response characteristics.

The wavelength of the light emitted by the light source 10 may be any wavelength contained in the wavelength range from 650 nm or higher to 950 nm or lower, for example. This wavelength range is included in the wavelength range from red to near-infrared. In this specification, the term "light" is used to refer not only to visible light but also infrared light. The above wavelength range is called the "biological window", and has the property of being absorbed less readily by the fluids and skin of a living body. In the case of treating a living body as the detection target, using light in the above wavelength range allows for higher detection sensitivity. In the case of detecting blood flow changes in the skin and the brain of the target person 500 like in the present embodiment, the light used is thought to be absorbed mainly by oxygenated hemoglobin (HbO₂) and deoxygenated hemoglobin (Hb). The wavelength dependency of light absorption is different between oxygenated hemoglobin and deoxygenated hemoglobin. Typically, if a change in blood flow occurs, the concentration of oxygenated hemoglobin and deoxygenated hemoglobin changes, and therefore the degree of light absorption also changes. Consequently, if the blood flow changes, the amount of light that is detected also changes over time.

The light source 10 may also emit light of two or more wavelengths contained in the above wavelength range. Such light of multiple wavelengths may also be respectively emitted from a plurality of light sources.

In the biometric apparatus 100 according to the present embodiment, because the target person 500 is measured in a non-contacting way, a light source 10 designed with consideration for the effects on the retina may be used. For example, a light source 10 that satisfies Class 1 of the laser safety standards formulated by various countries may be used. When Class 1 is satisfied, the target person 500 is irradiated with low-illumination light having an accessible exposure limit (AEL) below 1 mW. Note that the light source 10 itself does not have to satisfy Class 1. For example, Class 1 of the laser safety standards may still be satisfied by diffusing or attenuating the light with a component such as a diffuser or an ND filter disposed in front of the light source 10.

In the related art, a streak camera is used to distinguish and detect information such as an absorption coefficient or a scattering coefficient at different locations in the depth direction inside a living body. For example, Japanese Laid-open Patent Publication No. H4-189349 discloses an example of such a streak camera. With such a streak camera, to measure with the desired spatial resolution, ultra-short light pulses having a pulse width in femtoseconds or picoseconds are used.

In contrast, the biometric apparatus 100 of the present disclosure is capable of distinguishing and detecting the surface reflection component I1 and the internal scattering component I2. Consequently, the light emitted by the light source 10 does not have to be ultra-short light pulses, and the pulse width can be freely chosen.

In the case of irradiating the forehead with light to measure cerebral blood flow, the amount of light of the internal scattering component I2 may be an extremely small value several thousand times smaller or several tens of thousands of times smaller than the amount of light of the surface reflection component I1. Furthermore, if laser safety standards are considered, the amount of light that can be radiated becomes extremely small, and detection of the internal scattering component I2 becomes extremely difficult. Even in such cases, if the light source 10 is made to produce a light pulse having a relatively wide pulse width, the cumulative quantity of the internal scattering component I2 can be increased after a time lag. With this arrangement, the amount of detected light can be increased and the SN ratio can be improved.

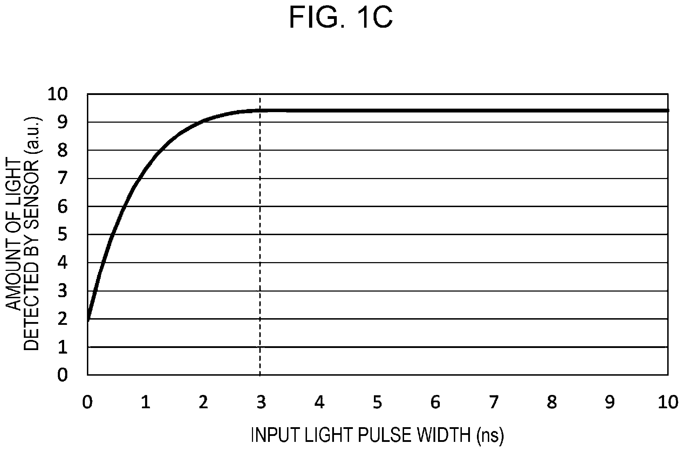

The light source 10 emits a light pulse having a pulse width of 3 ns or greater, for example. Typically, the temporal spreading of light scattered inside biological tissue such as the brain is approximately 4 ns. Fig. 1B illustrates an example of the change over time in the amount of light reaching the image sensor 20 in the case where the width of the input light pulse is 0 ns, 3 ns, and 10 ns, respectively. As illustrated in Fig. 1B, as the width of the light pulse from the light source 10 is widened, the amount of light of the internal scattering component I2 appearing in the trailing part of the light pulse returning from the target person 500 increases. Fig. 1C is a graph illustrating the width of an input light pulse on the horizontal axis and the amount of light detected by the sensor on the vertical axis. The image sensor 20 is provided with an electronic shutter. The result in Fig. 1C is obtained under the condition of opening the electronic shutter after 1 ns elapses from the time when the trailing edge of the light pulse is reflected at the surface of the target person 500 and reaches the image sensor 20. The reason for choosing this condition is that the ratio of the surface reflection component I1 to the internal scattering component I2 is high immediately after the trailing edge of the light pulse arrives. As illustrated in Fig. 1C, if the pulse width of the light pulse emitted by the light source 10 is set to 3 ns or more, the amount of light detected by the sensor can be maximized.

The light source 10 may also emit a light pulse having a pulse width of 5 ns or more, or even 10 ns or more. On the other hand, if the pulse width is too large, an increasing amount of light is unused and wasted. Accordingly, the light source 10 is made to produce a light pulse having a pulse width of 50 ns or less, for example. Alternatively, the light source 10 may emit a light pulse having a pulse width of 30 ns or less, or even 20 ns or less.

The irradiation pattern of the light source 10 may be a pattern having a uniform intensity distribution inside the irradiation area, for example. In this respect, the present embodiment differs from the biometric apparatus of the related art disclosed in literature such as Japanese Laid-open Patent Publication No. H11-164826, for example. In the apparatus disclosed in Japanese Laid-open Patent Publication No. H11-164826, the detector and the light source are separated by approximately 3 cm, and the surface reflection component is spatially separated from the internal scattering component. For this reason, discrete light irradiation must be used. In contrast, the biometric apparatus 100 according to the present embodiment is capable of temporally separating and reducing the surface reflection component I1 from the internal scattering component I2. For this reason, the light source 10 with an irradiation pattern having a uniform intensity distribution can be used. An irradiation pattern having a uniform intensity distribution may also be formed by using a diffuser to diffuse the light emitted by the light source 10.

In the present embodiment, unlike the technology of the related art, the internal scattering component I2 can be detected even directly beneath the irradiation point of the light pulse on the target person 500. By irradiating the target person 500 with light over a wide spatial range, the measurement resolution can also be increased.

### [1-2. Image sensor 20]

The image sensor 20 receives light emitted from the light source 10 and reflected or scattered by the target person 500. The image sensor 20 includes a plurality of photodetector cells disposed in a two-dimensional matrix, and acquires two-dimensional information about the target person 500 all at once. In this specification, the photodetector cells are also referred to as "pixels". For example, the image sensor 20 is an image sensor of any type, such as a CCD image sensor or a CMOS image sensor.

The image sensor 20 includes an electronic shutter. The electronic shutter is a circuit that controls the timing of imaging. In the present embodiment, the sensor controller 34 in the control circuit 30 has the function of an electronic shutter. The electronic shutter controls a single signal storage period in which received light is converted into a valid electrical signal and accumulated, and a period in which signal storage is stopped. The signal storage period may also be referred to as the "exposure period" or the "imaging period". In the following description, the width of the exposure period is also referred to as the "shutter width". The time from when a single exposure ends until the next exposure period begins is referred to as the "non-exposure period". Hereinafter, the state of exposure is referred to as the "OPEN" state, and the state of stopped exposure is referred to as the "CLOSE" state.

The image sensor 20 is capable of adjusting the exposure period and the non-exposure period with the electronic shutter in a sub-nanosecond range, such as from 30 ps to 1 ns, for example. To correct the influence of the brightness of the subject, a time of flight (TOF) camera of the related art for the purpose of distance measurement detects all returning light that is emitted from the light source 10 and reflected by the subject. Consequently, in a TOF camera of the related art, the shutter width must be larger than the light pulse width. In contrast, in the biometric apparatus 100 according to the present embodiment, it is not necessary to correct the amount of light from the subject. For this reason, the shutter width does not have to be larger than the pulse width. Therefore, the shutter width can be set to a value in the range from 1 ns or greater to 30 ns or less, for example. According to the biometric apparatus 100 of the present embodiment, because the shutter width can be reduced, the influence of dark current included in the detection signal can be reduced.

In the case of irradiating the forehead of the target person 500 with light and detecting information such as cerebral blood flow, the attenuation rate of light internally is extremely large. For example, the emitted light may be attenuated to approximately one millionth of the incident light. For this reason, to detect the internal scattering component I2, the amount of light from the irradiation of a single pulse may be insufficient in some cases. The amount of light radiated according to Class 1 of the laser safety standards is particularly weak. In this case, the sensitivity can be improved by accumulating the detection signal by having the light source 10 emit light pulses multiple times and correspondingly exposing the image sensor 20 multiple times with the electronic shutter.

Hereinafter, an exemplary configuration of the image sensor 20 will be described.

The image sensor 20 may be provided with a plurality of pixels arranged in a two-dimensional matrix on an imaging surface. Each pixel may be provided with a photoelectric converter such as a photodiode for example and one or a plurality of charge accumulators. Hereinafter, an example will be described in which each pixel is provided with a photoelectric converter that produces a signal charge corresponding to the amount of received light through photoelectric conversion, a charge accumulator that accumulates a signal charge produced by the surface reflection component I1 of the light pulse, and a charge accumulator that accumulates a signal charge produced by the internal scattering component I2 of the light pulse. In the following example, the control circuit 30 causes the surface reflection component I1 to be detected by detecting the portion of the light pulse returning from the head of the target person 500 before the light pulse starts to fall. The control circuit 30 also causes the internal scattering component I2 to be detected by detecting the portion of the light pulse returning from the head of the target person 500 after the light pulse starts to fall. The light source 10 is assumed to emit light of two different wavelengths.

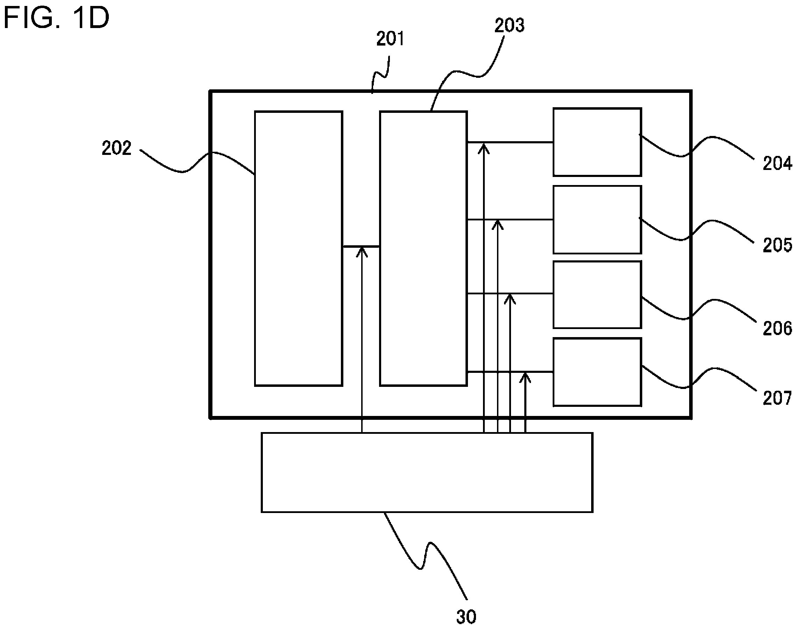

Fig. 1D is a diagram illustrating an example of a schematic configuration of a pixel 201 in the image sensor 20. Note that Fig. 1D schematically illustrates a configuration of a single pixel 201, and does not necessarily reflect the actual structure. The pixel 201 in this example includes a photodiode 203 that performs photoelectric conversion, a first floating diffusion 204, a second floating diffusion 205, a third floating diffusion 206, and a fourth floating diffusion 207 which are charge accumulators, and a drain 202 that flushes the signal charge.

Photons incident on each pixel originating from the emission of a single light pulse are converted into a signal charge, that is, signal electrons, by the photodiode 203. The converted signal electrons are flushed to the drain 202 or distributed to one of the first floating diffusion 204, the second floating diffusion 205, the third floating diffusion 206, and the fourth floating diffusion 207, according to a control signal input from the control circuit 30.

The emission of a light pulse from the light source 10, the storage of the signal charge in the first floating diffusion 204, the second floating diffusion 205, the third floating diffusion 206, and the fourth floating diffusion 207, and the flushing of the signal charge to the drain 202 are repeated in the above order. These repeating operations are performed at high speed, and may be repeated tens of thousands of times to hundreds of millions of times within the time of a single frame (for example, approximately 1/30 of a second) of a moving image, for example. Finally, the pixel 201 generates and outputs four image signals based on the signal charges accumulated in the first floating diffusion 204, the second floating diffusion 205, the third floating diffusion 206, and the fourth floating diffusion 207.

The control circuit 30 in this example causes the light source 10 to emit a first light pulse having a first wavelength and a second light pulse having a second wavelength repeatedly in order. By selecting two wavelengths with different absorptivity with respect to the internal tissue of the target person 500 as the first wavelength and the second wavelength, the state of the target person 500 can be analyzed. For example, a wavelength longer than 805 nm may be selected as the first wavelength, and a wavelength shorter than 805 nm may be selected as the second wavelength. With this arrangement, it is possible to detect changes in each of the oxygenated hemoglobin concentration and the deoxygenated hemoglobin concentration in the blood of the target person 500.

First, the control circuit 30 causes the light source 10 to emit the first light pulse. The control circuit 30 causes the first floating diffusion 204 to accumulate the signal charge in a first period during which the surface reflection component I1 of the first light pulse is incident on the photodiode 203. Thereafter, the control circuit 30 causes the second floating diffusion 205 to accumulate the signal charge in a second period during which the internal scattering component I2 of the first light pulse is incident on the photodiode 203. Next, the control circuit 30 causes the light source 10 to emit the second light pulse. The control circuit 30 causes the third floating diffusion 206 to accumulate the signal charge in a third period during which the surface reflection component I1 of the second light pulse is incident on the photodiode 203. Thereafter, the control circuit 30 causes the fourth floating diffusion 207 to accumulate the signal charge in a fourth period during which the internal scattering component I2 of the second light pulse is incident on the photodiode 203.

In this way, after the emission of the first light pulse is started, the control circuit 30 causes the signal charge from the photodiode 203 to be accumulated successively in the first floating diffusion 204 and the second floating diffusion 205, with a predetermined time difference in between. Thereafter, after the emission of the second light pulse is started, the control circuit 30 causes the signal charge from the photodiode 203 to be accumulated successively in the third floating diffusion 206 and the fourth floating diffusion 207, with a predetermined time difference in between. The above operations are repeated a plurality of times. To estimate the amounts of disturbance light and ambient light, a period of storing the signal charge in another floating diffusion not illustrated while the light source 10 is turned off may also be provided. By subtracting the signal charge quantity in the other floating diffusion from the signal charge quantities in the first floating diffusion 204, the second floating diffusion 205, the third floating diffusion 206, and the fourth floating diffusion 207, it is possible to obtain signals from which the disturbance light and ambient light component has been removed.

Note that in the present embodiment, there are four charge accumulators, but the number of charge accumulators may be set to a number equal to or greater than two depending on the purpose. For example, in the case of using only one wavelength, there may be two charge accumulators. Also, for an application that uses one wavelength and does not detect the surface reflection component I1, there may be one charge accumulator per pixel. Also, even in the case of using two or more wavelengths, if the imaging using each of the wavelengths is performed in different frames, there may be one charge accumulator. Also, as described later, if the detection of the surface reflection component I1 and the detection of the internal scattering component I2 are performed in different frames, there may be one charge accumulator.

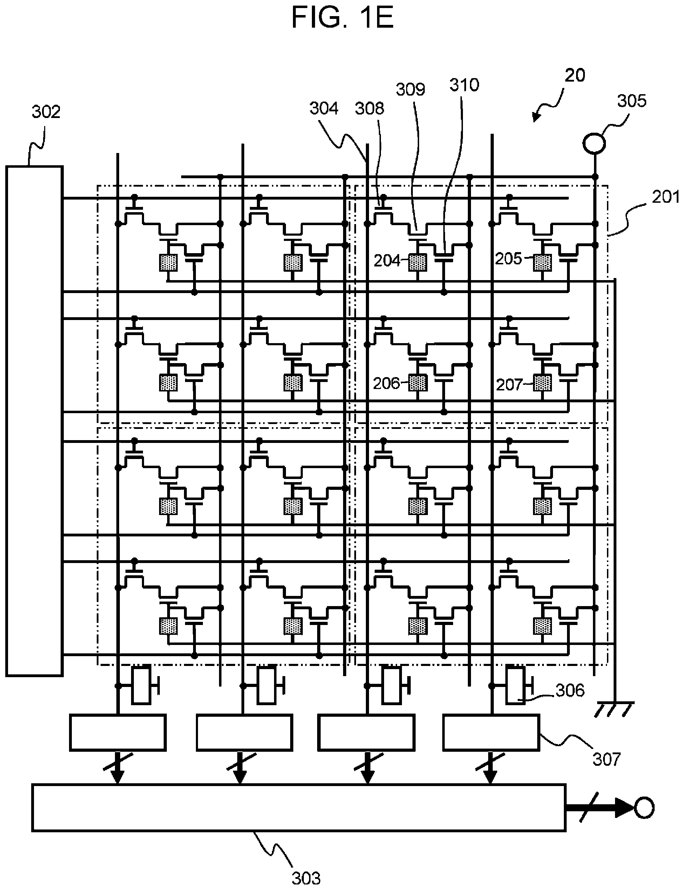

Fig. 1E is a diagram illustrating an example of the configuration of the image sensor 20. In Fig. 1E, the regions respectively enclosed by two-dot chain lines each correspond to a single pixel 201. Each pixel 201 includes one photodiode. In Fig. 1E, only four pixels arranged in a 2×2 matrix of rows and columns is illustrated, but an even greater number of pixels may be disposed in actuality. Each pixel 201 includes the first floating diffusion 204, the second floating diffusion 205, the third floating diffusion 206, and the fourth floating diffusion 207. The signals accumulated in the first floating diffusion 204, the second floating diffusion 205, the third floating diffusion 206, and the fourth floating diffusion 207 are treated as though the signals come from four pixels in a typical CMOS image sensor, and are output from the image sensor 20.

Each pixel 201 has four signal detection circuits. Each signal detection circuit includes a source follower transistor 309, a row select transistor 308, and a reset transistor 310. In this example, the reset transistor 310 corresponds to the drain 202 illustrated in Fig. 1D, and a pulse input into the gate of the reset transistor 310 corresponds to a drain discharge pulse. Each transistor is a fieldeffect transistor formed on a semiconductor substrate for example, but is not limited thereto. As illustrated in the diagram, the input terminal and one output terminal (typically the source) of the source follower transistor 309 is connected to the input terminal and one output terminal (typically the drain) of the row select transistor 308. The gate which is the control terminal of the source follower transistor 309 is connected to the photodiode 203. The signal charge (that is, holes or electrons) generated by the photodiode 203 is accumulated in the charge accumulator, namely the floating diffusion, between the photodiode 203 and the source follower transistor 309.

Although not illustrated in Fig. 1E, the first floating diffusion 204, the second floating diffusion 205, the third floating diffusion 206, and the fourth floating diffusion 207 are connected to the photodiode 203. Switches may be provided between the photodiode 203 and each of the first floating diffusion 204, the second floating diffusion 205, the third floating diffusion 206, and the fourth floating diffusion 207. The switches act in response to a signal storage pulse from the control circuit 30, and toggle the conduction state between the photodiode 203 and each of the first floating diffusion 204, the second floating diffusion 205, the third floating diffusion 206, and the fourth floating diffusion 207. With this arrangement, the starting and stopping of the signal charge storage in each of the first floating diffusion 204, the second floating diffusion 205, the third floating diffusion 206, and the fourth floating diffusion 207 are controlled. The electronic shutter in the present embodiment has a configuration for such exposure control.

The signal charges accumulated in the first floating diffusion 204, the second floating diffusion 205, the third floating diffusion 206, and the fourth floating diffusion 207 are read out by using a row select circuit 302 to turn on the gate of the row select transistor 308. At this time, the current flowing in from a source follower power supply 305 to the source follower transistor 309 and a source follower load 306 is increased according to the signal potential of the first floating diffusion 204, the second floating diffusion 205, the third floating diffusion 206, and the fourth floating diffusion 207. An analog signal expressed by the current read out from a vertical signal line 304 is converted into digital signal data by an analogdigital (AD) conversion circuit 307 connected to each column. The digital signal data is read out from each column by a column select circuit 303, and is output from the image sensor 20. After performing the readout of one row, the row select circuit 302 and the column select circuit 303 perform the readout of the next row, and thereafter read out the information of the signal charges in the floating diffusions on all rows in a similar way. After reading out all of the signal charges, the control circuit 30 turns on the gate of the reset transistor 310 to reset all of the floating diffusions. With this arrangement, the imaging of a single frame is completed. Thereafter, by repeating the high-speed imaging of frames in a similar way, the imaging of a series of frames by the image sensor 20 is finished.

Although the present embodiment describes an example of a CMOS image sensor 20, the image sensor 20 may also be an image sensor of another type. For example, the image sensor 20 may also be a CCD sensor, a single-photon counting sensor, or an amplifying image sensor (for example, an EMCCD sensor or an ICCD sensor).

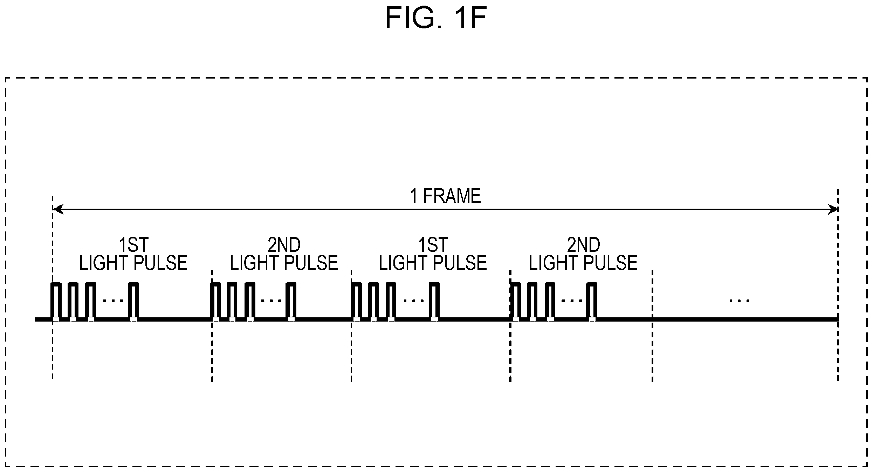

Fig. 1F is a diagram illustrating an example of operations in a single frame according to the present embodiment. As illustrated in Fig. 1F, in a single frame, the emission of the first light pulse and the emission of the second light pulse may be performed in alternation multiple times. With this arrangement, the time difference in the acquisition timings of detection images from two wavelengths can be reduced, making it possible to take images with the first and second light pulses at substantially the same time, even for a moving target person 500.

In the present embodiment, the image sensor 20 detects both the surface reflection component I1 and the internal scattering component I2. From temporal or spatial changes in the surface reflection component I1, first biological information about the target person 500 can be acquired. The first biological information may be the pulse of the target person 500, for example. On the other hand, from temporal or spatial changes in the internal scattering component I2, second biological information about the target person 500, namely brain activity information, can be acquired.

The first biological information may also be acquired by a method different from the method of detecting the surface reflection component I1. For example, the first biological information may be acquired by using a detector of another type different from the image sensor 20. In this case, the image sensor 20 detects only the internal scattering component I2. The detector of another type may be radar or thermography, for example.

The first biological information may be at least one of the pulse, perspiration, respiration, and body temperature of the target person, for example. It is sufficient for the first biological information to be biological information other than the brain activity information obtained by detecting the internal scattering component I2 of the light pulse irradiating the head of the target person. Herein, "other than the brain activity information" does not mean that the first biological information contains no information originating from brain activity whatsoever. It is sufficient for the first biological information to include biological information originating from biological activity other than brain activity. For example, the first biological information is biological information originating from autonomous or reflexive biological activity.

In this specification, a signal expressing the first biological information may be referred to as the "first biological signal" or simply the "biological signal". Also, a signal expressing brain activity information may be referred to as the "brain activity signal".

### [1-3. Control circuit 30 and signal processing circuit 40]

The control circuit 30 adjusts the time difference between the light pulse emission timing of the light source 10 and the shutter timing of the image sensor 20. Hereinafter, this time difference may be referred to as the "phase" or the "phase lag". The "emission timing" of the light source 10 refers to the timing when a light pulse emitted by the light source 10 begins to rise. The "shutter timing" refers to the timing when exposure begins. The control circuit 30 may adjust the phase by varying the emission timing or adjust the phase by varying the shutter timing.

The control circuit 30 may be configured to remove an offset component from the signal detected by each pixel of the image sensor 20. The offset component is a signal component due to ambient light such as sunlight or fluorescent light, or disturbance light. The offset component due to ambient light or disturbance light may be estimated by having the image sensor 20 detect a signal while the light source 10 is not emitting light, or in other words while the light source 10 is turned off.

The control circuit 30 may be an integrated circuit such as a microcontroller that combines a processor and memory or has a processor and memory built in, for example. By having the processor execute a program recorded in the memory for example, the control circuit 30 performs processes such as adjusting the emission timing and the shutter timing, estimating the offset component, and removing the offset component.

The signal processing circuit 40 is a circuit that processes the image signal output from the image sensor 20. The signal processing circuit 40 performs arithmetic processing such as image processing. The signal processing circuit 40 may be achieved by a digital signal processor (DSP) or a programmable logic device (PLD) such as a field-programmable gate array (FPGA), or by a combination of a central processing unit (CPU) or a graphics processing unit (GPU) and a computer program, for example. Note that the control circuit 30 and the signal processing circuit 40 may be a single combined circuit or separate, discrete circuits. In addition, the signal processing circuit 40 may also be a component of an external device such as a server provided in a remote location, for example. In this case, the external device such as a server is provided with a communication interface, and bidirectionally transmits and receives data with the light source 10, the image sensor 20, and the control circuit 30.

The signal processing circuit 40 in the present embodiment is capable of generating moving image data expressing change over time in the blood flow at the skin surface and cerebral blood flow on the basis of the signal output from the image sensor 20. The signal processing circuit 40 is not limited to such moving image data, and may also generate other information. For example, by synchronizing with other equipment, biological information such as the quantity of blood flow in the brain, the blood pressure, the blood oxygen saturation level, or the heart rate may also be generated.

Changes in the quantity of cerebral blood flow or blood components such as hemoglobin are known to have a close relationship with neural activity in human beings. For example, a change in the activity of nerve cells according to an emotional change in a human being causes the quantity of cerebral blood flow or the blood components to change. Consequently, if biological information such as changes in the quantity of cerebral blood flow or the blood components can be measured, the psychological state of a target person can be estimated. The psychological state of a target person means states such as mood (for example, pleasantness or unpleasantness), emotions (such as relief, anxiety, sadness, or anger, for example), state of health (for example, vitality or fatigue), and temperature sense (for example, hot, cold, or humid). The psychological state also includes indicators of the degree of brain activity derived from the above, such as a level of proficiency, level of learning, and a level of concentration, for example. The signal processing circuit 40 may also estimate the psychological state such as the level of concentration of a target person on the basis of changes in properties such as the quantity of cerebral blood flow, and output a signal expressing the estimation result.

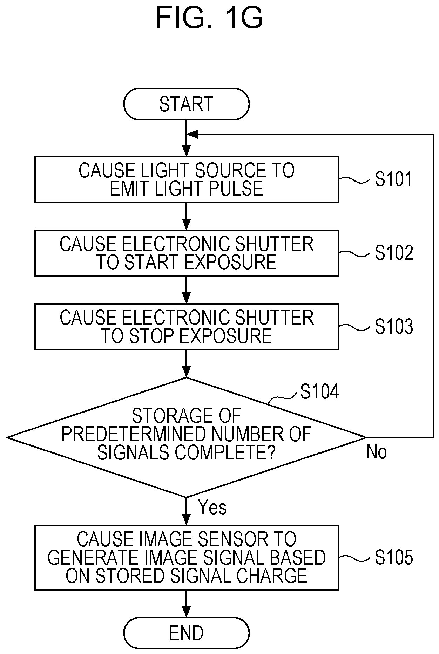

Fig. 1G is a flowchart illustrating an overview of operations by the control circuit 30. The control circuit 30 executes the operations schematically illustrated in Fig. 1G. Note that the operations described herein are for the case of detecting only the internal scattering component I2. First, the control circuit 30 causes the light source 10 to emit a light pulse for a predetermined time (step S101). At this time, the electronic shutter of the image sensor 20 is in the state of stopped exposure. The control circuit 30 causes the electronic shutter to stop exposure until the completion of the period during which a portion of the light pulse reflects off the surface of the target person 500 and reaches the image sensor 20. Next, at the timing when another portion of the light pulse is scattered inside the target person 500 and reaches the image sensor 20, the control circuit 30 causes the electronic shutter to start exposure (step S102). After a predetermined amount of time elapses, the control circuit 30 causes the electronic shutter to stop exposure (step S103). Next, the control circuit 30 determines whether or not the number of times that the above signal storage has been executed has reached a predetermined number of times (step S104). In the case of a No determination, steps S101 to S103 are repeated until a Yes determination is obtained. If a Yes determination is obtained in step S104, the control circuit 30 causes the image sensor 20 to generate and output a signal expressing an image based on the signal charge accumulated in each floating diffusion (step S105).

According to the above operations, the component of light scattered inside the measurement target can be detected with high sensitivity. Note that executing emission and exposure a plurality of times is not strictly necessary, and is executed as appropriate.

### [1-4. Other]

The biometric apparatus 100 may also be provided with an image-forming optical system that forms a two-dimensional image of the target person 500 on the light-receiving face of the image sensor 20. The optical axis of the image-forming optical system is substantially orthogonal to the light-receiving face of the image sensor 20. The image-forming optical system may also include a zoom lens. If the position of the zoom lens changes, the magnification of the two-dimensional image of the target person 500 is changed, and the resolution of the two-dimensional image on the image sensor 20 changes. Consequently, even if the distance to the target person 500 is far, it is possible to enlarge a desired region to measure and observe the region in detail.

Additionally, the biometric apparatus 100 may also be provided with a bandpass filter that passes only the band of the wavelength emitted from the light source 10 or nearby wavelengths, the bandpass filter being provided between the target person 500 and the image sensor 20. With this arrangement, the influence of the disturbance component such as ambient light can be reduced. The bandpass filter contains a multilayer filter or an absorption filter. In consideration of band shifts associated with the temperature of the light source 10 and oblique incidence on the filter, the bandwidth of the bandpass filter may be given a width approximately from 20 nm to 100 nm.

The biometric apparatus 100 may also be provided with a polarizer between the light source 10 and the target person 500, and also between the image sensor 20 and the target person 500. In this case, the polarization directions of the polarizer disposed near the light source 10 and the polarizer disposed near the image sensor exist in a crossed-Nicol relationship. With this arrangement, it is possible prevent the specular reflection component of the surface reflection component I1 of the target person 500, or in other words the component for which the angle of incidence is equal to the angle of reflection, from reaching the image sensor 20. In other words, the amount of light in the surface reflection component I1 that reaches the image sensor 20 can be reduced.

### [2. Operations]

The biometric apparatus 100 of the present disclosure is capable of distinguishing and detecting the internal scattering component I2 from the surface reflection component I1. In the case where the target person 500 is a human being and the target area is the forehead, the signal strength from the internal scattering component I2 to be detected is extremely weak. As described earlier, this is because in addition to irradiating the forehead with an extremely small amount of light that satisfies the laser safety standards, much of the light is scattered and absorbed by the scalp, cerebrospinal fluid, the cranium, gray matter, white matter, and blood flow. Furthermore, changes in the signal strength due to changes in the quantity of blood flow or the blood components during brain activity are several tens of times smaller. Consequently, in the present embodiment, an image is taken while limiting as much as possible the mixing-in of the surface reflection component I1, which is several thousands of times to several tens of thousands of times stronger than the desired signal component to be detected.

Hereinafter, an example of the operations of the biometric apparatus 100 according to the present embodiment will be described.

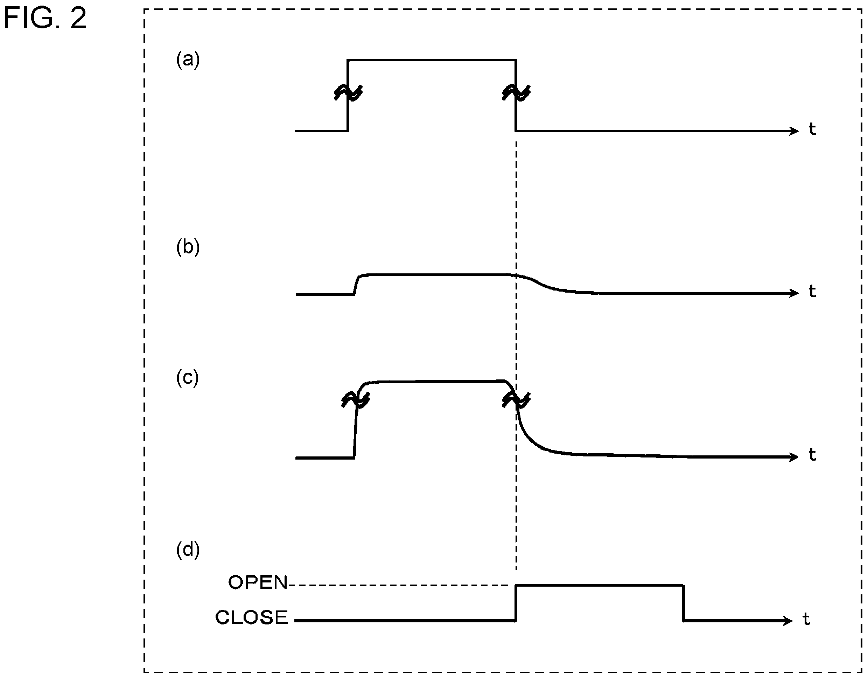

As illustrated in Fig. 1A, when the light source 10 irradiates the target person 500 with a light pulse, the surface reflection component I1 and the internal scattering component I2 are produced. A portion of each of the surface reflection component I1 and the internal scattering component I2 reaches the image sensor 20. Compared to the surface reflection component I1, the internal scattering component I2 has a longer optical path length from being emitted from the light source 10 until reaching the image sensor 20, because the internal scattering component I2 passes through the interior of the target person 500. Consequently, the internal scattering component I2 reaches the image sensor 20 later on average than the surface reflection component 11. Fig. 2 is a diagram illustrating optical signals obtained when a square light pulse is emitted from the light source 10, and light returning from the target person 500 reaches the image sensor 20. For each of the waveforms (a) to (d), the horizontal axis represents time (t), while the vertical axis represents strength for the waveforms (a) to (c). For the waveform (d), the vertical axis represents the OPEN or CLOSE state of the electronic shutter. The waveform (a) expresses the surface reflection component I1. The waveform (b) expresses the internal scattering component I2. The waveform (c) expresses the sum component of the surface reflection component I1 and the internal scattering component I2. As the waveform (a) indicates, the surface reflection component I1 maintains a square shape. On the other hand, as the waveform (b) indicates, the internal scattering component I2 is the sum of light traveling a variety of optical path lengths, and therefore exhibits a characteristic of a drawn-out trailing edge of the light pulse (in other words, the falling time is longer than that of the surface reflection component I1). To extract a higher ratio of the internal scattering component I2 from the optical signal exhibiting the waveform (c), it is sufficient to cause the electronic shutter to start exposure from the trailing edge of the surface reflection component I1 (in other words, on or after the time when the surface reflection component I1 falls), as the waveform (d) indicates. The shutter timing is adjusted by the control circuit 30. As described above, because it is sufficient for the biometric apparatus 100 according to an embodiment of the present disclosure to be able to distinguish and detect the surface reflection component I1 and the internal scattering component I2 that reaches into a deep part of the target, the light pulse width and the shutter width may be any values. Consequently, unlike the method of the related art that uses a streak camera, the biometric apparatus 100 can be realized with a simple configuration, and costs can be greatly reduced.

In the waveform (a) in Fig. 2, the trailing edge of the surface reflection component I1 falls perpendicularly. In other words, the time from the start to the end of the falling of the surface reflection component I1 is zero. However, in reality, the rising portion and the falling portion of the waveform of the light pulse itself radiated by the light source 10 are not completely perpendicular, tiny irregularities exist on the surface of the target person 500, or scattering occurs inside the epidermis, and as a result, the trailing edge of the surface reflection component I1 does not fall perpendicularly in some cases. Also, because the target person 500 is an opaque body, the amount of light in the surface reflection component I1 is much larger than the internal scattering component I2. Consequently, there is a possibility that the internal scattering component I2 will be buried, even in cases where the trailing edge of the surface reflection component I1 slightly sticks out past the perpendicular falling position. Also, because of a time lag associated with electron movement during the readout period of the electronic shutter, an ideal binary readout like that illustrated by the waveform (d) in Fig. 2 may not be achievable in some cases. Consequently, the control circuit 30 may delay the shutter timing of the electronic shutter until slightly after the falling of the surface reflection component I1. For example, the shutter timing may be delayed approximately from 0.5 ns to 5 ns. Note that instead of adjusting the shutter timing of the electronic shutter, the control circuit 30 may also adjust the emission timing of the light source 10. It is sufficient for the control circuit 30 to adjust the time difference between the shutter timing of the electronic shutter and the emission timing of the light source 10. In the case of measuring changes in the quantity of blood flow or the blood components during brain activity in a non-contacting way, if the shutter timing is delayed too much, the internal scattering component I2 which is small to begin with decreases even further. For this reason, the shutter timing may be kept in the vicinity of the trailing edge of the surface reflection component I1. Because the time lag due to scattering by the target person 500 is 4 ns, the maximum amount of delay in the shutter timing is approximately 4 ns.

The amount of light detected from the internal scattering component I2 may also be amplified by having the light source 10 emit a plurality of light pulses and performing exposure a plurality of times at shutter timings in phase with each of the light pulses.

Note that instead of or in addition to disposing a bandpass filter between the target person 500 and the image sensor 20, the control circuit 30 may also estimate the offset component by taking an image with the same exposure time while putting the light source 10 in a non-emitting state. The estimated offset component is removed by being subtracted from the signal detected by each pixel of the image sensor 20. With this arrangement, the dark current component produced in the image sensor 20 can be removed.

The internal scattering component I2 contains information about the internal characteristics of the target person 500, such as cerebral blood flow information, for example. The amount of light absorbed by the blood changes according to temporal fluctuations in the quantity of cerebral blood flow in the target person 500, and the amount of light detected by the image sensor 20 also increases or decreases correspondingly. Consequently, monitoring the internal scattering component I2 makes it possible to estimate the brain activity state from changes in the quantity of cerebral blood flow in the target person 500. In this specification, among the signals output from the image sensor 20, the signal expressing the internal scattering component I2 may be referred to as the "brain activity signal". The brain activity signal may include information about the increase or decrease of cerebral blood flow in the target person.

Next, an example of a method of detecting the surface reflection component I1 will be described. The surface reflection component I1 contains information about the surface characteristics of the target person 500, such as information about the blood flow in the face and the scalp, for example. The image sensor 20 detects the surface reflection component I1 from the optical signal obtained when a light pulse emitted by the light source 10 reaches the target person 500 and returns to the image sensor 20.

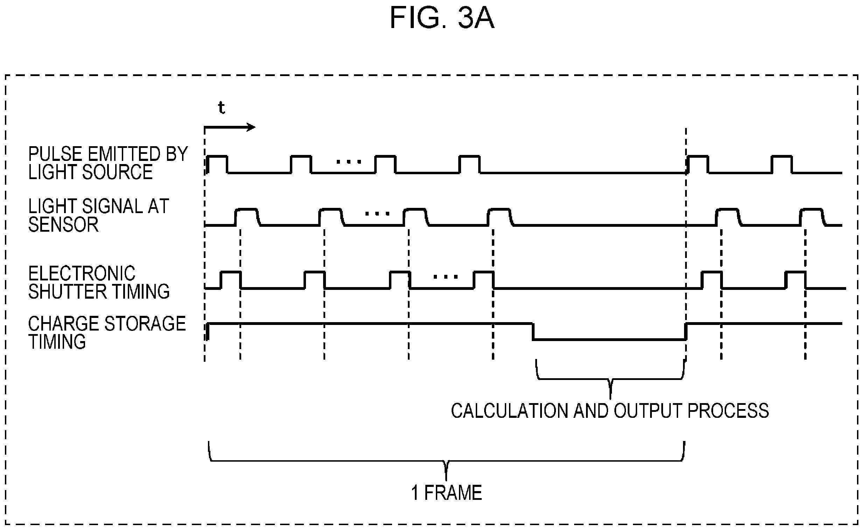

Fig. 3A illustrates an example of a timing chart in a case of detecting the surface reflection component I1. As illustrated in Fig. 3A for example, to detect the surface reflection component I1, the shutter may be opened before the light pulse reaches the image sensor 20, and closed before the trailing edge of the light pulse reaches the image sensor 20. By controlling the shutter in this way, the mixing-in of the internal scattering component I2 can be reduced. The ratio of light transmitted near the surface of the target person 500 can be increased. Particularly, the timing for closing the shutter may be set to immediately after the light reaches the image sensor 20. With this arrangement, signal detection with a higher ratio of the surface reflection component I1 having a relatively short optical path length is possible. Acquiring the signal of the surface reflection component I1 makes it possible to detect the pulse or the oxygen concentration of facial blood flow in the target person 500. Other methods of acquiring the surface reflection component I1 may include the image sensor 20 acquiring the entire light pulse, or detecting the radiation of continuous light from the light source 10.

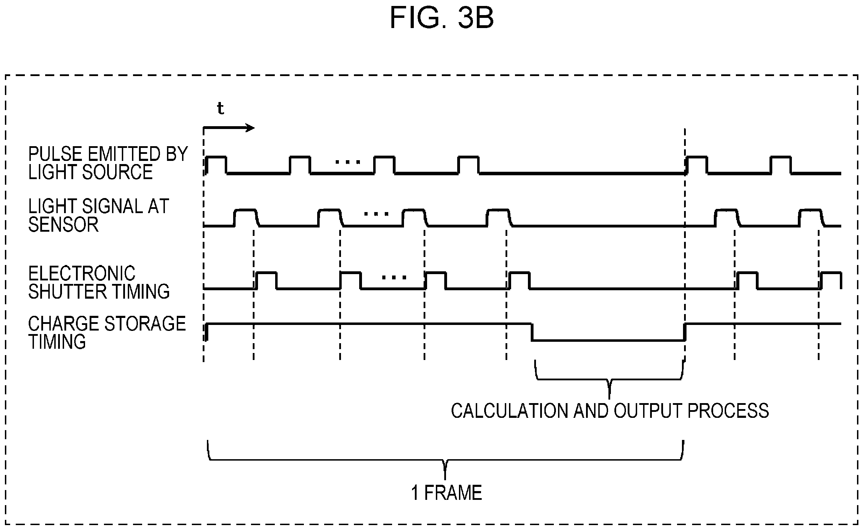

Fig. 3B illustrates an example of a timing chart in a case of detecting the internal scattering component I2. By opening the shutter in the period when the trailing edge portion of the pulse reaches the image sensor 20, the signal of the internal scattering component I2 can be acquired.

The surface reflection component I1 may also be detected by an apparatus other than the biometric apparatus 100 that acquires the internal scattering component I2. The apparatus that acquires the internal scattering component I2 may be a separate apparatus, or a separate device such as a pulsimeter or a Doppler blood flow monitor may be used. In the case of using a separate device, the device is used in consideration of timing synchronization between devices, optical interference, and calibration of the detection sites. If time-division imaging using the same camera or the same sensor is performed like in the present embodiment, spatial and temporal inconsistencies occur less readily. In the case of acquiring the signals of both the surface reflection component I1 and the internal scattering component I2 with the same sensor, the component to acquire may be alternated every frame, as illustrated in Figs. 3A and 3B. Alternatively, as described with reference to Figs. 1D to 1F, the component to acquire may be alternated rapidly inside a single frame. In this case, the detection time difference between the surface reflection component I1 and the internal scattering component I2 can be reduced.

Additionally, the respective signals of the surface reflection component I1 and the internal scattering component I2 may be acquired using two wavelengths of light. For example, light pulses with the two wavelengths of 750 nm and 850 nm may be used. With this arrangement, changes in the concentration of oxygenated hemoglobin and changes in the concentration of deoxygenated hemoglobin can be computed from the changes in the amount of detected light for each wavelength. In the case of acquiring the surface reflection component I1 and the internal scattering component I2 with two respective wavelengths, a method of rapidly alternating between four types of charge storage inside a single frame may be used, as described with reference to Figs. 1D to 1F. According to such a method, temporal inconsistencies in the detection signals can be reduced.

The biometric apparatus 100 irradiates the forehead of the target person 500 with pulses of near-infrared light or visible light, and is capable of detecting changes in the quantity of oxygenated hemoglobin in the scalp or face, or the pulse, from temporal changes in the surface reflection component I1. The light source 10 for acquiring the surface reflection component I1 emits near-infrared light or visible light. If near-infrared light is used, measurement can be performed at any time of day or night. In the case of measuring the pulse, visible light having higher sensitivity may be used. If measurement is performed during the day, disturbance light, namely sunlight or an indoor light source, may be used instead of illumination. In cases where the amount of light is insufficient, the illumination may be augmented with a special-purpose light source. The internal scattering component I2 includes the light component that reaches the brain. By measuring the change over time of the internal scattering component I2, temporal increases and decreases in the quantity of cerebral blood flow can be measured.

Because the light reaching the brain is also transmitted through the scalp and the surface of the face, fluctuations of blood flow in the scalp and the face are also detected and superposed on the signal. To eliminate or reduce this influence, the signal processing circuit 40 may perform a process of subtracting the surface reflection component I1 from the internal scattering component I2 detected by the image sensor 20. With this arrangement, pure cerebral blood flow information excluding blood flow information about the scalp and the face can be acquired. For the subtraction method, a method may be used in which a value obtained by multiplying the signal of the surface reflection component I1 by one or more coefficients decided in consideration of differences in optical path lengths is subtracted from the signal of the internal scattering component I2, for example. The one or more coefficients may be found by simulation or experiment on the basis of an average value of the cranial optical constants of typical persons, for example. Such a subtraction process is particularly easy to execute in the case of performing measurement using the same camera or sensor and using the same wavelength of light. This is because temporal and spatial inconsistencies are easily reduced, and it is easy to match the scalp blood flow component contained in the internal scattering component I2 to the characteristics of the surface reflection component I1.

The cranium exists between the brain and the scalp. For this reason, a two-dimensional distribution of the cerebral blood flow and a two-dimensional distribution of the blood flow in the scalp and the face are independent. Consequently, a two-dimensional distribution of the internal scattering component I2 and a two-dimensional distribution of the surface reflection component I1 may also be separated on the basis of signals detected by the image sensor 20 by using a statistical method such as independent component analysis or principal component analysis.

Next, an example of using the biometric apparatus 100 above to perform vital sensing of the target person 500 will be described.

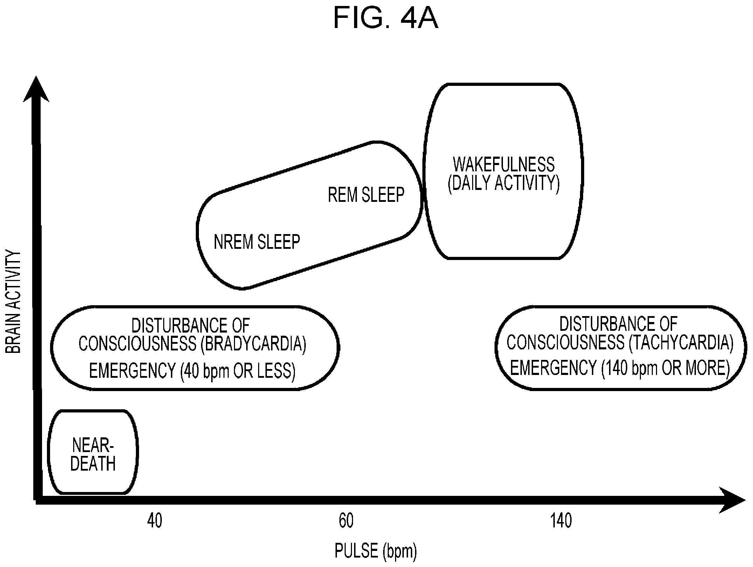

Fig. 4A is a diagram schematically illustrating the relationship between a person's pulse, brain activity, and bodily state. In Fig. 4A, the horizontal axis represents the pulse in beats per minute, while the vertical axis represents brain activity. Brain activity is estimated according to the magnitude of change in the signal strength of the internal scattering component I2. For example, the brain activity can be estimated according to an indicator such as the difference between the maximum value and the minimum value or the time rate of change of the signal strength of the internal scattering component I2.

When a person is awake, both the pulse and the brain activity are relatively large. On the other hand, when a person is asleep, the brain activity does not change very much, but the pulse is lowered compared to when awake. Consequently, by computing the pulse from the measurement data of the surface reflection component I1, it can be distinguished whether the target person 500 is awake or asleep.

During sleep, the cerebral cortex works strongly to organize and consolidate memories. In contrast, during times of disturbed consciousness, brain activity is lowered. Consequently, by measuring changes in the quantity of cerebral blood flow from the measurement data of the internal scattering component I2, it is possible to distinguish whether the target person 500 is asleep or in a state of disturbed consciousness. Regarding the pulse in particular, there are regions of overlap between wakefulness, sleep, and disturbed consciousness, and distinguishing between these states is difficult using pulse information alone. Disturbed consciousness includes the case of bradycardia and the case of tachycardia. Bradycardia is a state in which the pulse is lower than normal, and induces disturbed consciousness because the supply of blood to the brain is insufficient. The state is considered dangerous if the pulse falls to 40 bpm or below. On the other hand, tachycardia is a state in which the pulse is extremely rapid. The state is considered dangerous if the pulse rises to 140 bpm or above. Disturbed consciousness in the case of bradycardia is difficult to distinguish from sleep. On the other hand, disturbed consciousness in the case of tachycardia is difficult to distinguish from wakefulness. By also utilizing information about changes in the quantity of cerebral blood flow like in the present embodiment, more accurate discrimination of states is possible.

Also, because brain activity is lowered both during times of mindfulness and during times of disturbed consciousness, it is difficult to distinguish between a state of mindfulness and a state of disturbed consciousness by measuring the quantity of cerebral blood flow alone. However, during times of mindfulness, the pulse is closer to a normal value compared to times of disturbed consciousness, and therefore by measuring the pulse in addition to measuring the quantity of cerebral blood flow, it is possible to distinguish between a state of mindfulness and a state of disturbed consciousness.

The oxygen consumption of the brain is approximately 20% of the body's total oxygen consumption, and the cerebral blood flow is approximately 15% of the body's total blood flow. If the cerebral blood flow is lowered, brain activity falls, and consciousness is disturbed. The center of cognition resides in the cerebral cortex. For this reason, the biometric apparatus 100 observes changes in the cerebral blood flow in the cerebral cortex through the skin of the forehead on the exposed head. With this arrangement, it is possible to distinguish whether a person is in a state of disturbed consciousness, an awake state, or sleeping state.

The biological information that may be used in the present embodiment is not limited to the pulse, and may also be other information, such as perspiration, body temperature, or respiration, for example. By using brain activity information, it is possible to appropriately distinguish between bodily states that are difficult to distinguish using perspiration, body temperature, or respiration alone.

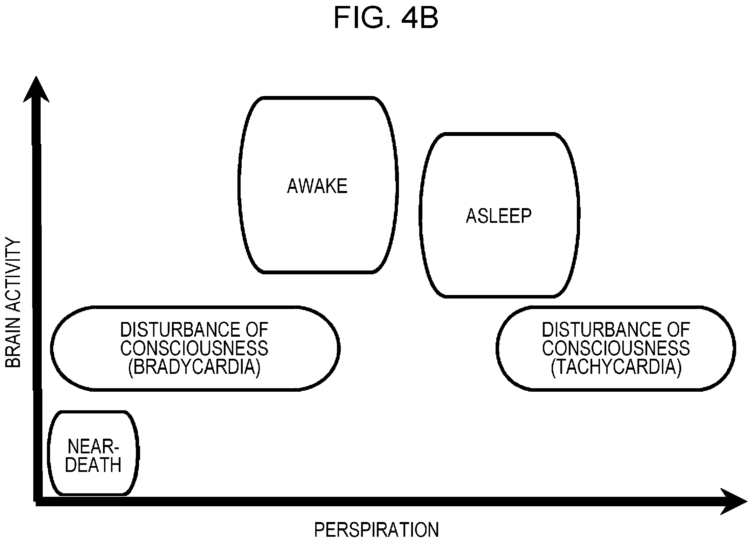

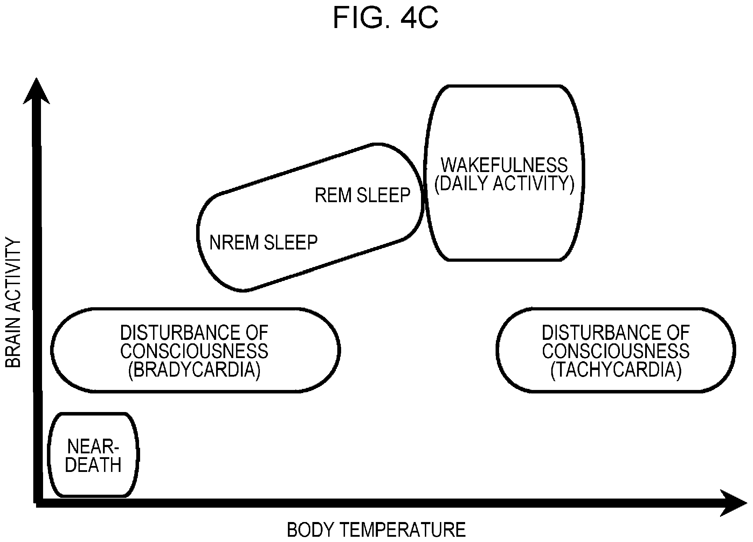

Fig. 4B is a diagram schematically illustrating the relationship between a person's perspiration, brain activity, and bodily state. Fig. 4C is a diagram schematically illustrating the relationship between a person's body temperature, brain activity, and bodily state. During sleep and in the cases of both REM sleep and NREM sleep, perspiration is greater and body temperature is lower compared to wakefulness. Typically, at the start of sleep, a large perspiration effect occurs, the body temperature drops rapidly, and the person enters NREM sleep. After approximately one hour, the body temperature rises and the brain enters REM sleep, which is a state close to wakefulness. After that, perspiration occurs again and the body temperature also falls, and the person transitions to NREM sleep. This cycle is repeated multiple times during sleep.

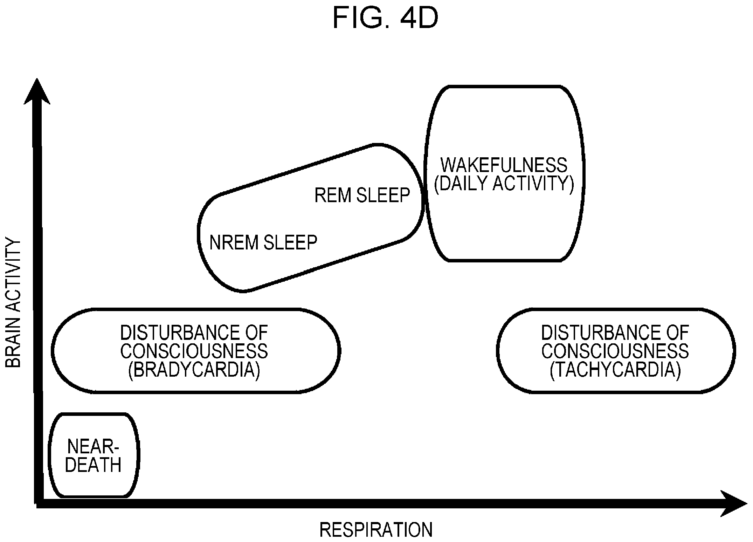

Fig. 4D is a diagram schematically illustrating the relationship between a person's respiration, brain activity, and bodily state. In REM sleep, respiration is lower compared to wakefulness. In NREM sleep, respiration decreases further by 10% to 0%. Also, during bradycardia, respiration is reduced compared to a calm state, and during tachycardia, respiration tends to increase to compensate for insufficient oxygen.

As illustrated in Figs. 4B to 4D, similarly to pulse, it is possible to distinguish whether a person is in a sleeping state or an awake state according to perspiration, body temperature, or respiration. However, it is difficult to distinguish whether a person is in a state of disturbed consciousness or a sleeping state with the above biological information along. By using brain activity information in addition to the above biological information like the present embodiment, it is possible to distinguish whether or not a person is in a state of disturbed consciousness.

In the case where the biometric apparatus 100 measures perspiration as the biological information, the biometric apparatus 100 may be provided with a sensor that measures perspiration. In the case where the biometric apparatus 100 measures body temperature as the biological information, the biometric apparatus 100 may be provided with a sensor that measures body temperature. In the case where the biometric apparatus 100 measures respiration as the biological information, the biometric apparatus 100 may be provided with a sensor that measures respiration. These sensors may be image sensors that detect infrared light, for example. Perspiration, respiration, or body temperature may also be measured by the image sensor 20 that acquires brain activity information. Also, image processing may be used to estimate perspiration, body temperature, or respiration. In this way, the biological signal may include information about at least one of pulse, perspiration, respiration, and body temperature.

Next, an application of the biometric apparatus 100 according to the present embodiment will be described.

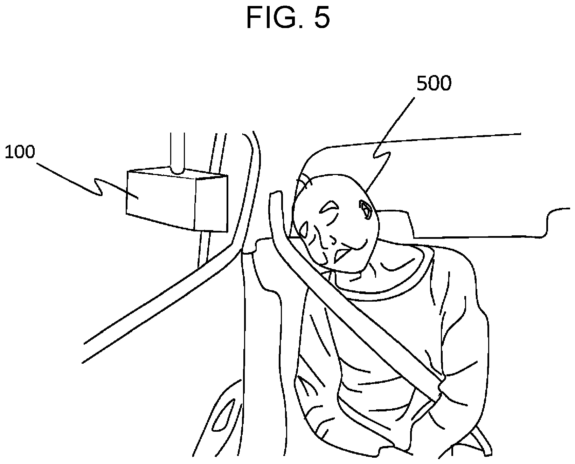

Fig. 5 illustrates how the vital signs of a passenger in a self-driving taxi are detected by the biometric apparatus 100. The biometric apparatus 100 is installed inside the cabin. The biometric apparatus 100 performs vital sensing of the passenger when picking up or while carrying the passenger by self-driving. If an abnormality of the passenger is detected, the biometric apparatus 100 uses wireless communication for example to notify an external apparatus, such as a server computer at a medical institution, about the abnormality of the passenger.

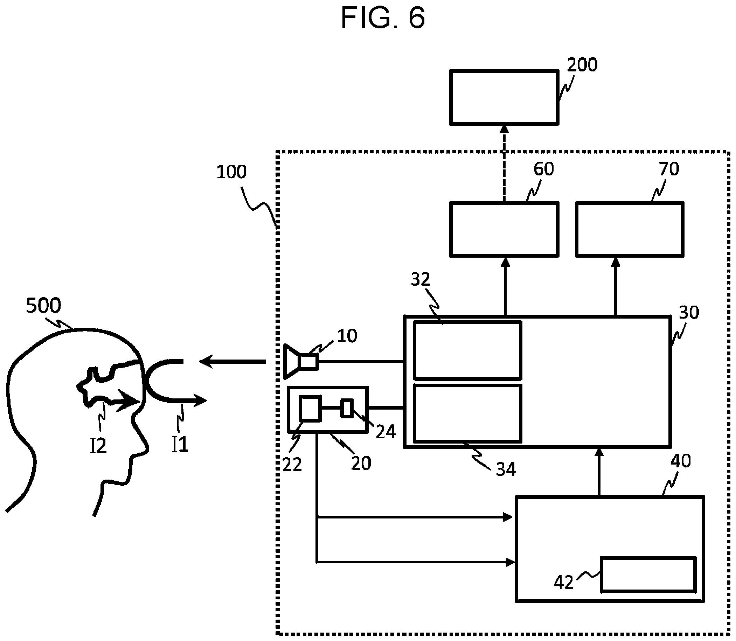

Fig. 6 is a diagram illustrating an exemplary configuration of the biometric apparatus 100 in this application. In this example, the biometric apparatus 100 is provided with a communication circuit 60 and a speaker 70 connected to the control circuit 30. The communication by the communication circuit 60 conforms to a publicly known wireless communication standard, for example. If the signal processing circuit 40 detects an abnormality of the passenger, namely the target person 500, the control circuit 30 notifies an external apparatus 200 at a medical institution or the like about the abnormality through the communication circuit 60.

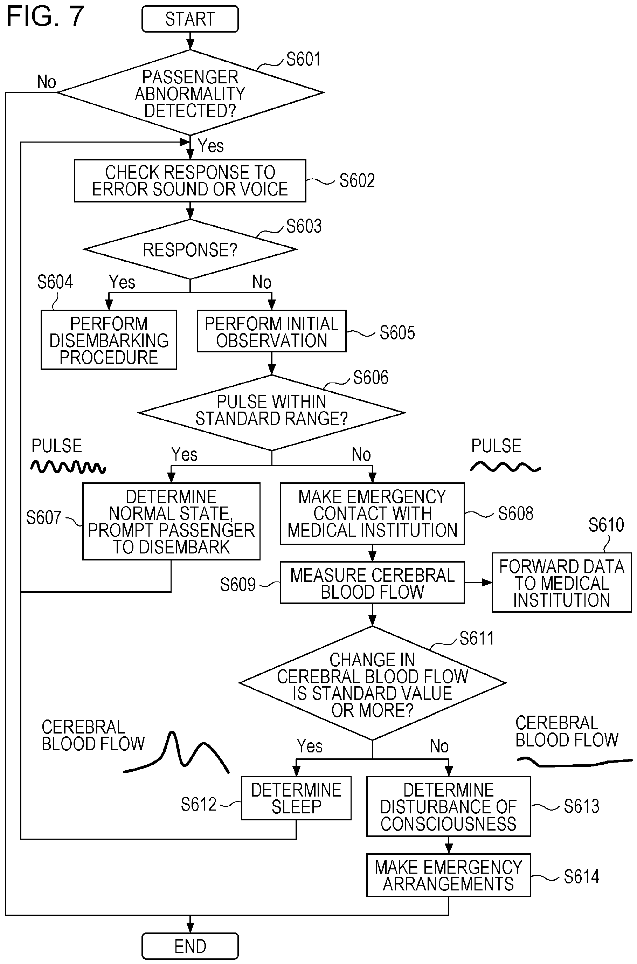

Fig. 7 is a flowchart illustrating exemplary operations by the biometric apparatus 100. When picking up or while carrying a passenger by self-driving, the control circuit 30 of the biometric apparatus 100 determines whether there is an abnormality of the passenger (step S601). For example, an abnormality is determined to exist if the passenger is acting strangely or if the passenger does not exit the vehicle even after a fixed amount of time has elapsed since arriving at the destination. The determination may be made on the basis of output from the image sensor 20 or another sensor not illustrated, for example. In the case where an abnormality of the passenger is detected, first, a call-out to the passenger using an error sound or speech is made to check the condition of the passenger (step S602). The call-out is made through the speaker 70. Note that the speaker 70 may also be installed in the vehicle rather than in the biometric apparatus 100.

Next, the control circuit 30 determines whether there is a response from the passenger (step S603). The determination may be made on the basis of information such as an image acquired by the image sensor 20 or the voice of the passenger detected by a microphone not illustrated, for example. In the case where there is a response and no particular problem exists, after arriving at the destination, a payment process is performed by automatic or electronic payment, and the passenger exits the vehicle (step S604).

In the case where the passenger does not respond to the sound-based call-out, or in the case where the passenger seems to be moaning or groaning, there is a possibility that the passenger is asleep or in a state of disturbed consciousness. Accordingly, the biometric apparatus 100 performs an initial observation (step S605). First, the biometric apparatus 100 makes an automatic adjustment to point the light source 10 and the image sensor 20 of the biometric apparatus 100 toward the passenger. For example, the control circuit 30 adjusts the direction of the light source 10 and the image sensor 20 such that a light pulse from the light source 10 is incident on the passenger's forehead. The biometric apparatus 100 is provided with a driving mechanism such as a motor that makes such adjustment possible. In this state, the biometric apparatus 100 performs a respiration check, a check of the blood oxygen saturation level (SpO₂) based on the surface blood flow in the face and scalp, and a check of the pulse of the passenger, for example. SpO₂ is the ratio of the quantity of hemoglobin bonded to oxygen with respect to the total quantity of hemoglobin in the blood. The biometric apparatus 100 also has the function of a camera that acquires images. For this reason, respiration can be checked from the body movement of the passenger appearing in a camera image. To compute SpO₂, a measurement using two wavelengths of near-infrared light or a measurement using a red wavelength and a near-infrared wavelength may be performed. By calculating the ratio of the respectively detected amounts of light or the ratio of HbO₂ and Hb, SpO₂ can be estimated. The pulse can be measured from blood flow changes in the face or forehead of the passenger. The measurement may be performed over a short time, such as 10 seconds or 20 seconds for example. Basic vital sign data is acquired by such short-time screening.

Next, the control circuit 30 determines whether or not the measured value of the passenger's pulse is inside a standard range (step S606). In the case where the measured value is inside the standard range, the control circuit 30 determines that the passenger is asleep in a normal state, and prompts the passenger to exit the vehicle (step S607). For example, the control circuit 30 prompts the passenger to exit the vehicle using sound emitted from the speaker 70. The standard range of the pulse may be the range from 50 bpm or higher to 100 bpm or lower, for example.

In the case where the measured value of the pulse is outside the standard range, although there is a possibility that the pulse has dropped sharply during sleep, there is also a possibility of disturbed consciousness. For this reason, the biometric apparatus 100 immediately contacts a medical institution (step S608). For example, the control circuit 30 transmits a signal or an alert indicating that the passenger's pulse is an abnormal value to a computer at a medical institution through the communication circuit 60. At this time, information such as an ID and the location of the vehicle for example may also be transmitted as well. The biometric apparatus 100 starts measuring the cerebral blood flow (step S609).

Because it takes time for emergency personnel to reach mountainous locations and remote communities particularly, there is demand for an environment capable of checking the passenger's vital signs immediately to allow for quick decision-making. The cerebral blood flow may be measured by having the biometric apparatus 100 irradiate the passenger's head with light and detecting the internal scattering component I2 scattered by the frontal lobe. If the passenger is in a state of disturbed consciousness, there is a high probability that the variations in cerebral blood flow are greatly reduced compared to the sleeping state. For this reason, it is possible to distinguish whether the passenger is asleep or in a state of disturbed consciousness on the basis of measured variations in the cerebral blood flow.

The cerebral blood flow measurement time in step S609 is longer than the pulse measurement time in step S605. The reason for this is that the period of change in cerebral blood flow is relatively long compared to the relatively short period of the pulse. The cerebral blood flow may be measured over a time from 1 minute or more to 5 minutes or less, for example. The control circuit 30 forwards the data obtained by the measurement to an external apparatus 200 such as a computer at a medical institution such as a hospital in real-time, for example (step S610). With this arrangement, a physician can check the passenger's vital signs immediately. The biometric apparatus 100 is also capable of acquiring a near-infrared image or a visible-light image, and therefore may also transmit such an image. This arrangement makes it possible to observe the condition of the passenger through the image. Also, in step S610, a signal of the pulse may also be transmitted as well.

Next, the signal processing circuit 40 of the biometric apparatus 100 determines whether the amount of change in the cerebral blood flow is a standard value or greater (step S611). The amount of change in the cerebral blood flow may be a value such as the difference between the maximum value and the minimum value of the quantity of cerebral blood flow in a single period, the amplitude of the cerebral blood flow, or the time rate of change of the cerebral blood flow, for example. In the case where the value is smaller than a predetermined standard value, the variation in the cerebral blood flow is determined to be small, and the passenger is determined to be in a state of disturbed consciousness (step S613). In this case, the control circuit 30 transmits a signal requesting emergency arrangements to the external apparatus 200 through the communication circuit 60 (step S614).

As above, in the present embodiment, the signal processing circuit 40 performs an initial observation as a first diagnosis and a measurement of the cerebral blood flow as a second diagnosis in order. In the initial observation, the signal processing circuit 40 estimates the state of the target person on the basis of a biological signal different from a brain activity signal, such as the pulse for example, and outputs a first signal indicating the estimation result. In the cerebral blood flow measurement, the signal processing circuit 40 estimates the state of the target person 500 on the basis of a brain activity signal, and outputs a second signal indicating the estimation result. In the present embodiment, the second diagnosis takes more time than the first diagnosis. In other words, the time from the start of irradiation with a light pulse until the output of the brain activity signal is longer than the time from the start of irradiation with a light pulse until the acquisition of the biological signal. Also, the time from the acquisition of the brain activity signal to the output of the second signal is longer than the time from the acquisition of the biological signal such as the pulse to the output of the first signal. In the case where the signal processing circuit 40 determines that the target person 500 is not in a normal state on the basis of the above biological signal and/or brain activity signal, the communication circuit 60 notifies the external apparatus 200 at a medical institution. The notification may include the first and second signals.

According to operations like the above, actions such as dispatching an ambulance to the scene can be executed quickly, even in the case where a passenger aboard a self-driving vehicle suffers a disturbance of consciousness. Like the above example, in cases where the passenger is asleep or unconscious, there is little body movement. For this reason, a necessary brain activity signal can be acquired without having to correct the positions of the light source and the detector because of body movement.

In the example of Fig. 7, there is a possibility that a problem with the passenger's health may exist even if the passenger recovers consciousness during the course of observation. For this reason, a remote medical interview or whole-body observation may also be performed. A remote medical interview or whole-body observation can be performed by contact between a physician and the passenger through the camera of the biometric apparatus 100. For example, if the physician strongly suspects that the passenger is experiencing low blood sugar, the physician may check whether the passenger is able to clench his or her hands, or check the passenger's eyes (for example, the pupils, eye position and eye movement, or nystagmus). Alternatively, if the physician strongly suspects a stroke, the physician may check whether the passenger is experiencing a strong headache, nausea, dizziness, visual impairment, or aphasia.

The biometric apparatus 100 may also forward video data from the initial stages of the abnormality to the external apparatus 200. With this arrangement, a physician is able to check the progress of the disturbance of consciousness over time (such as continuation or recovery, for example). Also, the biometric apparatus 100 is not limited to being used during self-driving and when the passenger exits the vehicle, and is also valid when checking the passenger's vital signs at the time of an accident by self-driving. Unlike a cerebral blood flow measuring device of the related art, such as device utilizing functional near-infrared spectroscopy (fNIRS), the biometric apparatus 100 is capable of non-contacting measurement using a camera that includes the image sensor 20. For this reason, it is unnecessary to attach a device to the target person 500, and measurement is possible even in situations where no one other than the target person is present.

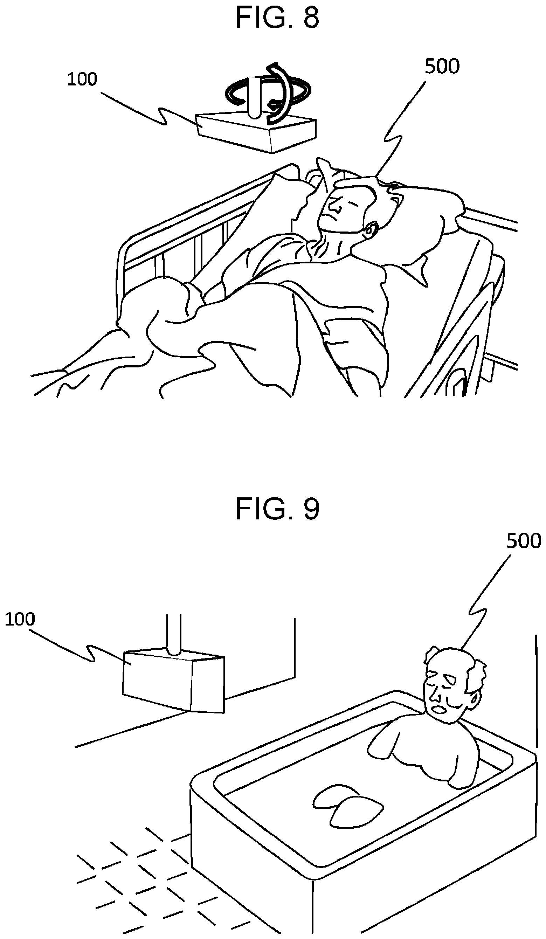

The checking of vital signs using the biometric apparatus 100 is not limited to the cabin of a vehicle, and may also be performed in another environment. For example, as illustrated in Fig. 8, biological information about a bedridden patient may also be measured. Also, as illustrated in Fig. 9, biological information about a person taking a bath may also be measured. The biometric apparatus 100 is deployable in any environment where it is desirable to check vital signs.

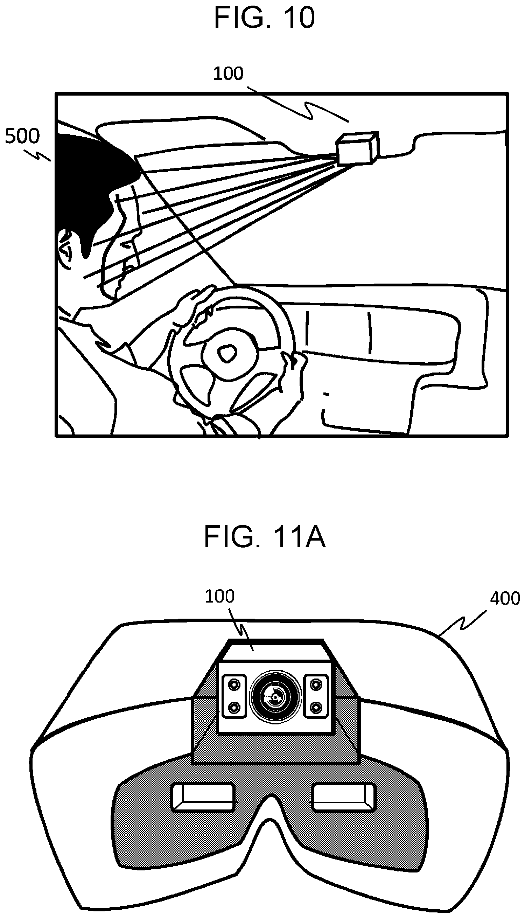

Fig. 10 is a diagram illustrating how the biometric apparatus 100 detects a disturbance of consciousness or drowsiness in the driver of a vehicle treated as the target person 500. The biometric apparatus 100 measures the pulse of the driver on the basis of the surface reflection component 11, and measures changes in the cerebral blood flow of the driver on the basis of the internal scattering component I2. On the basis of the acquired pulse data and cerebral blood flow data, it is determined whether the driver is in an awake state, a drowsy state, or a state of disturbed consciousness. In the cerebral blood flow measurement, the measured data is compared to a database of the quantity of cerebral blood flow or the cerebral blood flow distribution of the driver when driving normally, or to a database of the quantity of cerebral blood flow or the cerebral blood flow distribution of the driver in an awake state stored daily, and it is determined whether an abnormal value exists.

Additionally, perspiration may be measured instead of the pulse from the surface reflection component I1. The perspiration can be measured by the specular reflection component reflecting off the skin surface. If perspiration is present, the specular reflection component reflecting off the skin surface of the driver in the light radiated from the biometric apparatus 100 increases, and appears as a shiny component. The shiny component is detected from the image as a portion of high contrast compared to the surroundings. With this arrangement, perspiration due to tachycardia may be detected. By making an integrated evaluation of the cerebral blood flow data and the perspiration data, it is possible to improve the accuracy of determining whether the driver is in a state of disturbed consciousness or a drowsy state.

By sensing the cabin temperature, the state of direct sunlight, or the figure of the driver in parallel along with perspiration, it is possible to increase the accuracy of discerning whether the perspiration is due to the environment inside the cabin or due to the poor condition of the driver. The cabin temperature, the state of direct sunlight, and the figure of the driver may be detected using separate sensors attached to the interior of the cabin. Additionally, the state of direct sunlight and the figure of the driver may also be detected by utilizing the image sensor 20 provided in the biometric apparatus 100 as a camera. Additionally, by considering factors such as the path of travel detected by a separate sensing system attached to the vehicle body, or the speed of the vehicle compared to other nearby cars, it is possible to detect the state of the driver with even higher accuracy.

In the case of inferring that the driver is in a state of disturbed consciousness on the basis of the biological information such as skin blood flow information, and pulse information as well as the cerebral blood flow information, the biometric apparatus 100 makes a sound-based call-out to the driver. After performing the sound-based call-out, the state of the driver is detected again by the biometric apparatus 100. If the driver is again inferred to be in a state of disturbed consciousness, a medical institution or the like is contacted. In the case where a vehicle is provided with a self-driving system, a procedure of bringing the vehicle to a stop on the shoulder may also be executed.

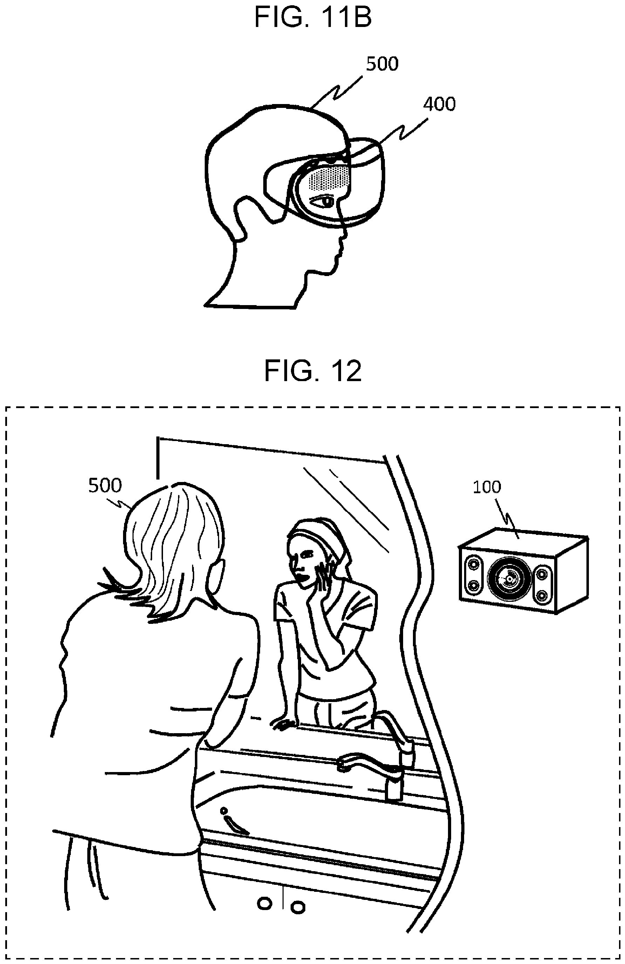

Fig. 11A illustrates an example of the biometric apparatus 100 integrated into a head-mounted display 400. Fig. 11B is a diagram illustrating how a target person uses the biometric apparatus 100 illustrated in Fig. 11A. The head-mounted display 400 is used for entertainment activities such as watching a movie or playing a video game. In the case of detecting a rise in pulse from the pulse information acquired by detecting the surface reflection component I1, there is a possibility that the target person is in a state of tension or feeling fear, whereas in the case of detecting a drop in pulse, there is possibility that the target person is in a relaxed state or a bored state. Also, if perspiration exists, there is a possibility that the target person is in a state of tension or an excited state. On the other hand, from the cerebral blood flow information acquired by detecting the internal scattering component I2, the level of understanding, level of concentration, or level of relaxation in the user treated as the target person 500 can be evaluated. By integrating the above information, it is possible to improve the certainty of estimating the psychological state of the user. Regarding the method of estimating the psychological state, it is sufficient to adapt machine learning to the integrated information combining the biological information acquired by detecting the surface reflection component I1 and the brain activity information acquired by detecting the internal scattering component I2. On the basis of the estimated psychological state, the image displayed on the head-mounted display 400 may be changed, the story may be changed, or the volume may be adjusted for example to adjust the intensity of the stimulation imparted to the user. As an example of volume adjustment, the contrast of the volume may be increased in the case of estimating that the content is not sufficiently scary.

In addition, the head-mounted display 400 may be applied to virtual reality (VR) or to applications that blend the real world with a virtual world, such as augmented reality (AR) or mixed reality (MR). For example, in cases such as providing navigation assistance using AR or issuing instructions for factory work, the user's level of understanding may be determined on the basis of the biological information acquired by detecting the surface reflection component I1 and the brain activity information acquired by detecting the internal scattering component I2, and repeat playback or an adjustment of the speed of announcements may be performed depending on the level of understanding.

Fig. 12 illustrates an example of using the biometric apparatus 100 for a daily health check by installing the biometric apparatus 100 inside a home or an office. Because the biometric apparatus 100 is capable of acquiring biological information and brain activity information in a non-contacting way, the biometric apparatus 100 may be installed behind a washstand mirror, for example. The mirror is treated with an interference film coating that reflects visible light while transmitting near-infrared light (for example, light having a wavelength of 700 nm or higher). By coating the mirror with an interference film that reflects visible light, the mirror reflects an image of the user treated as the target person 500, but the camera installed behind the mirror is not visible to the user. On the other hand, because the mirror transmits near-infrared light, it is possible to irradiate the user with measurement light through the mirror. Consequently, because the user is not made aware of the existence of the camera, the biological information and the cerebral blood flow information can be measured while the user is in a natural mental state. Measurement is also possible from the front of the user, and the head can be measured efficiently. Furthermore, because the user often uses a hair band to hold back the hair in front when washing his or her face, it is not necessary to instruct the user to hold up his or her hair that acts as an obstacle, leaving the user's hands free. Also, because the user stands in front of the mirror at the washstand, measurement at a fixed distance and at short range is possible, and the consistency of measurement is increased.

A presence sensor such as a pyroelectric sensor may also be installed near the biometric apparatus 100. If the presence sensor detects a person, a signal is transmitted to the biometric apparatus 100, and the measurement of the person by the biometric apparatus 100 is started. By cooperating with the presence sensor, the driving of the light source and the electrical circuits in the biometric apparatus 100 can be stopped when a person is not present, thereby reducing power consumption.

On the basis of the surface blood flow or pulse measured by the biometric apparatus 100, it is possible to acquire physical condition data such as a lack of sleep, fatigue, or anemia. Also, from the cerebral blood flow acquired at the same time, it is possible to detect mental condition data such as a state of concentration, the degree of mental acuity, or a depressed mood. In this way, by evaluating both the biological information acquired by detecting the surface reflection component I1 and the brain activity information acquired by detecting the internal scattering component I2, it is possible to estimate both a physical and a mental state of health.

The measurement by the biometric apparatus 100 may also be performed every time the user stands in front of the mirror, and the acquired data may be stored. From information about relative changes obtained by comparing the stored data from day to day, a condition on a certain day that is unusual with respect to the daily normal state of the user may be detected. Also, by accumulating data measurements in a daily time series and taking a moving average over time of the time-series data, high-frequency components expressing transient, irregular changes in condition can be removed, and the low-frequency components expressing long-term changes in conditions in units of multiple years can be monitored. As a result, such measurement may lead to the early detection of serious illnesses affecting the circulatory system or the nervous system, such as stroke, a brain tumor, or dementia. In this case, calibration may also be performed by pre-estimating the influence of seasonally variable components in atmospheric temperature and condition. By removing the influence of seasonally variable components in atmospheric temperature or condition, the detection accuracy can be improved.

In the case where the system detects a change in condition, the estimated state of health of the user is displayed on a display installed in the washstand or a display provided on the reflective mirror. Also, by outputting the daily measurement results as a graph, it is possible to visualize the degree of improvement in one's health and increase the user's motivation to actively improve his or her own healthcare. As another method of displaying data, the measurement data may be transmitted to a smartphone or tablet used by the user through Wi-Fi (registered trademark), Wi-Fi Direct (registered trademark), Bluetooth (registered trademark), the cloud, or the like, and the measurement data may be displayed on the smartphone or tablet.

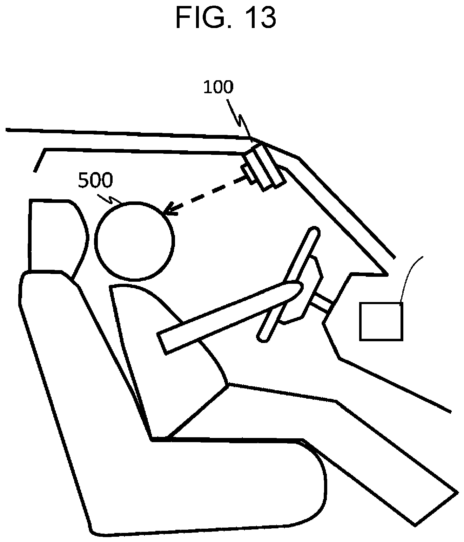

Fig. 13 is a diagram illustrating an example of applying the biometric apparatus 100 to detecting alcohol consumption by a driver treated as the target person 500. From the brain activity information acquired on the basis of the internal scattering component I2, it is evaluated whether the driver is capable of thinking rationally, whether his or her mind is sharp, and whether the driver is able to drive. Additionally, the quantity of surface blood flow, the pulse, or the perspiration is detected on the basis of the surface reflection component I1. By integrating the above information to make an evaluation, the probability that the driver has consumed alcohol is determined, and in the case where a high probability is determined, the driver is informed by sound or by displaying information on a display. Additionally, vehicle control is performed to block the engine from running, stop the engine, or the like.

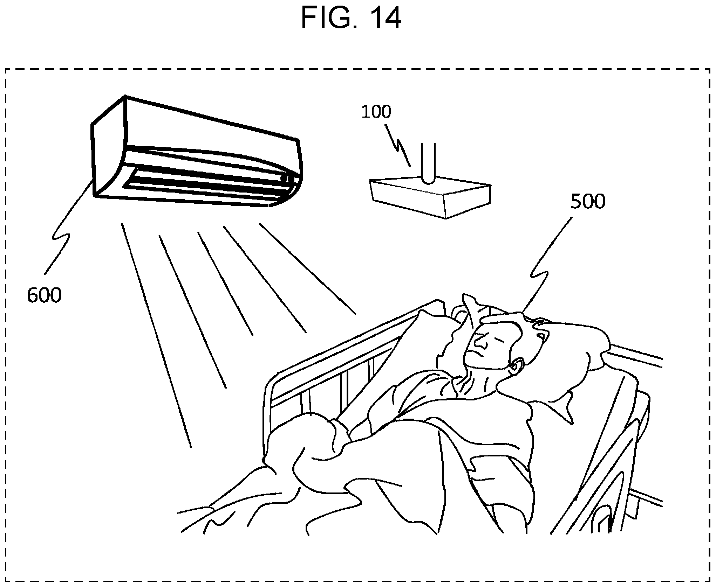

Fig. 14 is a diagram illustrating an example of using the biometric apparatus 100 to adjust indoor air conditioning. The perspiring state of a user may be detected both the information of both the surface reflection component I1 and the internal scattering component I2. Because the surface reflection component I1 largely contains the specular reflection component reflecting off the skin surface of the user, the surface reflectance rises as the user perspires, and can be detected as a shiny component. Also, the internal scattering component I2 is used to measure whether the user feels uncomfortably hot. When perspiration is detected from the surface reflection component I1 and a feeling of being uncomfortably hot is detected from the internal scattering component I2, the temperature, airflow, or direction of an air conditioning apparatus 600 may be controlled.

As above, the biometric apparatus 100 is capable of any of outputting a notification to the target person 500, controlling equipment near the target person 500, and communicating with an external apparatus on the basis of the result of a determination about the state of the target person 500. The notification to the target person includes applying visual, aural, tactile, or other stimulation to the target person. The equipment near the target person includes equipment is installed near the target person that may apply visual, aural, tactile, or other stimulation to the target person. The equipment near the target person also includes equipment that is operated by the target person. As described earlier, communicating with an external apparatus includes transmitting a signal or data to an apparatus such as a server installed in a location distant from the biometric apparatus 100.

In the present embodiment, as illustrated in Fig. 6, the signal processing circuit 40 is provided with memory 42, and a plurality of standard values determined on the basis of the data illustrated in Figs. 4A to 4D are stored in the memory 42. The signal processing circuit 40 compares a signal expressing the first biological information to a first standard value, and compares a signal expressing the brain activity information to a second standard value. With this arrangement, it is possible to determine the state of the target person on the basis of both of the signals expressing the first biological information and the brain activity information. In this way, the state of the target person may be determined by respectively comparing the signal expressing the first biological information and the signal expressing the brain activity information to two standard values stored in the memory 42.

The signal processing circuit 40 may also store a data table indicating the relationship between brain activity, activity related to the first biological information, and bodily state in the memory 42. The signal processing circuit 40 may also determine the state of the target person by referencing the data table to evaluate the acquired signal expressing the brain activity information and the acquired signal expressing the first biological information. In this way, the state of the target person may be determined by referencing the data table stored in the memory 42 to evaluate the biological signal and the brain activity signal. The data table expresses the relationship between the brain activity signal, the biological signal, and the bodily state.

The signal processing circuit 40 may also acquire time-series variation in the signal expressing the brain activity information and the signal expressing the first biological information. Additionally, the signal processing circuit 40 may also compare variation patterns or feature values of the time-series variation to reference patterns or reference feature values stored in the memory 42. Alternatively, the state of the target person may be determined by computing a statistical value such as correlation. Feature values of the time-series variation may be computed by loading a data set of a large number of target persons in advance and using supervised machine learning such as deep learning, a support-vector machine, a regression tree, or a Bayes estimator to learn features contained in the data set. The data set is a set of detection signals and correct values expressing the state of the target person. The current state of the target person may also be determined on the basis of machine learning treating feature values learned in advance as the learning data. Every time a target person is measured, the learning data may be updated using the new target person data. In some cases, as a result of a detailed examination by a specialized institution such as a medical institution, the determination result obtained by executing the flow in Fig. 7 for example is different from the true value. In this case, the correct value of the state of the target person may be corrected and machine learning may be reapplied.

Also, besides using supervised learning, the state of the target person may also be determined by using unsupervised machine learning such as hierarchical clustering, k-means clustering, a self-organizing map, or deep learning.

Besides time-series variation in the signal expressing the brain activity information and the signal expressing the first biological information, in the case of using a photodetector that functions as a camera or an image sensor, two-dimensional data may also be used as a data set. For example, on the basis of the distribution, bias, or positions of high intensity in a cerebral blood flow signal or the first biological signal in two-dimensional image data, the state of the target person may be determined, patterns or correlations may be compared, feature values may be extracted, or machine learning may be performed.

As above, the state of the target person may be determined by comparing reference patterns or reference feature values stored in the memory 42 to time-series data or the two-dimensional pattern, computing a statistical value, and/or using machine learning.

The signal processing circuit 40 may also determine the age, sex, or state of health of the target person on the basis of information related to the target person acquired by biometric apparatus 100 or another sensor, or on the basis of information related to the target person registered in advance. Additionally, on the basis of the determination result, the signal processing circuit 40 may update the standard values, the data table, and the reference patterns.

As above, according to an embodiment of the present disclosure, information related to the brain activity state of a target person can be measured without contacting the target person and in a state in which noise due to the reflection component reflecting off the surface of the target person is suppressed. Furthermore, the vital signs of the target person can be checked consistently by an inexpensive method.

### (Other embodiments)

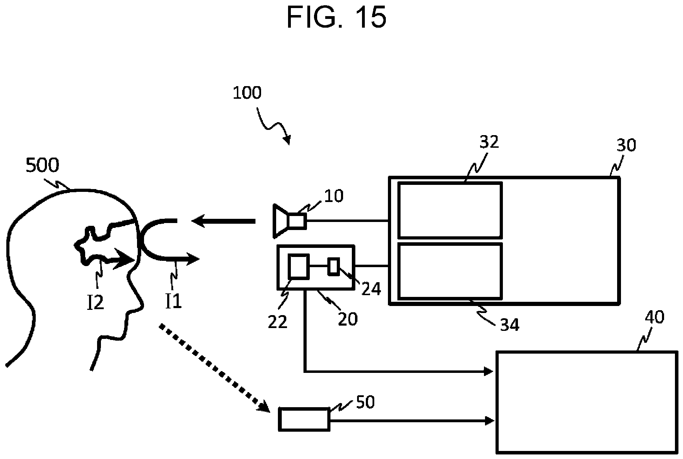

The biometric apparatus 100 may also acquire other biological information besides brain activity information from another detector different from a photodetector such as the image sensor 20. Fig. 15 is a flowchart illustrating an example of such a biometric apparatus 100. The biometric apparatus 100 in this example is provided with a detector 50 that measures biological information such as pulse, separately from the image sensor 20. If a millimeter-wave radar is used as the detector 50 for example, the pulse wave or respiration of the target person 500 can be detected. If a thermographic instrument is used as the detector 50 for example, the perspiration and body temperature of the target person 500 can be measured. In the case of using the detector 50, the image sensor 20 does not have to detect the surface reflection component I1 of the target person 500. On the basis of biological information about the target person 500 detected by the detector 50, the vital signs of the target person 500 can be checked.

The foregoing embodiment mainly describes an example in which the photodetector is the image sensor 20. However, the present disclosure is not limited to such an example. A combination of an avalanche photodiode and memory or a combination of a PIN photodiode and memory for example may also be used as the photodetector. Even if a combination of an avalanche photodiode and memory or a combination of a PIN photodiode and memory is used as the photodetector, by detecting the rising component and the falling component of a reflected light pulse for every pulse and repeatedly accumulating the detected components in the memory, for example, a sufficient signal quantity is obtained. By performing arithmetic operations using the signal quantity stored in the memory, a signal expressing the skin blood flow and a signal expressing the cerebral blood flow can be separated.

The present disclosure includes not only the biometric apparatus 100 but also a method of acquiring biological information using the biometric apparatus 100, and a vehicle provided with the biometric apparatus 100.

### Industrial Applicability

The biometric apparatus according to the present disclosure is useful in a camera or measuring equipment that acquires internal information about a target person in a non-contacting way. The biometric apparatus can be applied to biological or medical sensing.

### Reference Signs List

10 light source
20 image sensor
22 photoelectric converter
24 charge accumulator
30 control circuit
32 light source controller
34 sensor controller
40 signal processing circuit
42 memory
50 detector
60 communication circuit
70 speaker
100 biometric apparatus
200 external apparatus
400 head-mounted display
500 target person
600 air conditioning apparatus

## Claims

1. A biometric apparatus comprising:
a first detector (20) configured to detect and output a brain activity signal indicating a state of brain activity in a target person (500); and
a signal processing circuit (40), wherein the signal processing circuit (40) is configured to:
- acquire the brain activity signal,
- acquire a biological signal of the target person (500) that is different from the brain activity signal,
- determine, based on the biological signal and the brain activity signal a state of the target person (500), and
- generate and output a signal indicating the state of the target person (500);
wherein the brain activity signal includes information on a change in a quantity of cerebral blood flow in the target person (500); and
the signal processing circuit (40) is configured to:
- determine, based on the biological signal, whether the biological signal is within a standard range;
- determine, based on the brain activity signal, whether an amount of change in the cerebral blood flow is smaller than a standard value; and
- in response to the determination that the biological signal is within the standard range, determine that the state of the target person (500) is a normal state;
**characterized in that**: the signal processing circuit (40) is configured to:
- in response to the determination that the biological signal is not within the standard range and the determination that the amount of change in the cerebral blood flow is smaller than the standard value, determine that the state of the target person (500) is a state of disturbed consciousness, the target person (500) being unconscious during the state of disturbed consciousness; and
- in response to the determination that the biological signal is not within the standard range and the determination that the amount of change in the cerebral blood flow is not smaller than the standard value, determine that the state of the target person (500) is a sleeping state.

2. The biometric apparatus according to claim 1, wherein the first detector (20) is further configured to detect and output the biological signal.

3. The biometric apparatus according to claim 1, further comprising: a second detector (20, 50) configured to detect and output the biological signal.

4. The biometric apparatus according to any of claims 1 to 3, further comprising:
a light source (10) configured to emit a light pulse irradiating a head of the target person (500); and
a control circuit (30), wherein
the first detector (20) is configured to detect at least a part of a reflected pulse returning from the head, and
the control circuit (30) is configured to:
cause the light source (10) to emit the light pulse, and
cause the first detector (20) to detect and output an internal scattering component scattered inside a brain of the target person (500) from the reflected light pulse as the brain activity signal.

5. The biometric apparatus according to any of claims 1 to 4, wherein the biological signal includes at least one type of information selected from the group consisting of pulse, perspiration, respiration, and body temperature.

6. The biometric apparatus according to any of claims 1 to 5, wherein the signal processing circuit (40) is configured to acquire the brain activity signal after acquiring the biological signal.

7. The biometric apparatus according to any of claims 1 to 6, wherein the signal processing circuit (40) is configured to determine and output whether a pulse of the target person (500) is bradycardia or tachycardia, based on the biological signal and the brain activity signal.

8. The biometric apparatus according to any of claims 1 to 7, wherein the biological signal includes information on a blood flow at a surface of a skin of the target person (500).

9. The biometric apparatus according to claim 4, wherein
the control circuit (30) is configured to cause the first detector (20) to further detect a surface reflection component reflected at a surface of a skin of the target person (500) from the reflected light pulse, and
output a signal indicating a variation in the surface reflection component as the biological signal.

10. The biometric apparatus according to claim 9, wherein
the control circuit (30) is configured to cause the first detector (20) to detect the internal scattering component by detecting a portion of the reflected light pulse after an intensity of the reflected light pulse starts to decrease, and
detect the surface reflection component by detecting a portion of the reflected light pulse before the intensity of the reflected light pulse starts to decrease.

11. The biometric apparatus according to claim 4, wherein a time from a start of irradiation with the light pulse until an acquisition of the brain activity signal is longer than a time from the start of irradiation with the light pulse until an acquisition of the biological signal.

12. The biometric apparatus according to any of claims 1 to 11, wherein
the signal processing circuit (40) is configured to:
make a first determination related to the state of the target person (500) based on the biological signal, and output a first signal indicating a result of the first determination, and
after outputting the first signal, makes a second determination related to the state of the target person (500) based on the brain activity signal, and outputs a second signal indicating a result of the second determination.

13. The biometric apparatus according to claim 12, further comprising:
a communication circuit (60) configured to communicate with an external apparatus (200) external to the biometric apparatus, wherein
when the signal processing circuit (40) determines that the target person (500) is not in a normal state based on at least one selected from the group consisting of the biological signal and the brain activity signal, the communication circuit (60) notifies the external apparatus (200) that the target person (500) is not in the normal state.

## Patentansprüche

1. Biometrische Vorrichtung, die umfasst:
einen ersten Detektor (20), der so ausgeführt ist, dass er ein Gehirntätigkeits-Signal registriert und ausgibt, das einen Zustand von Gehirntätigkeit bei einer Zielperson (500) anzeigt; sowie
eine Signalverarbeitungs-Schaltung (40), wobei
die Signalverarbeitungs-Schaltung (40) ausgeführt ist zum:
Erfassen des Gehirntätigkeits-Signals,
Erfassen eines biologischen Signals der Zielperson (500), das sich von dem Gehirntätigkeits-Signal unterscheidet,
Feststellen eines Zustandes der Zielperson (500) auf Basis des biologischen Signals und des Gehirntätigkeits-Signals, sowie
Erzeugen und Ausgeben eines Signals, das den Zustand der Zielperson (500) anzeigt;
wobei das Gehirntätigkeits-Signal Informationen über eine Veränderung eines Maßes von zerebralem Blutfluss der Zielperson (500) einschließt; und
die Signalverarbeitungs-Schaltung (40) ausgeführt ist zum:
Feststellen, ob das biologische Signal innerhalb eines Standardbereiches liegt, auf Basis des biologischen Signals,
Feststellen, ob ein Betrag von Veränderung des zerebralen Blutflusses unter einem Standardwert liegt, auf Basis des Gehirntätigkeits-Signals, sowie
Feststellen, dass der Zustand der Zielperson (500) ein normaler Zustand ist, in Reaktion auf die Feststellung dahingehend, dass das biologische Signal innerhalb des Standardbereiches liegt,
**dadurch gekennzeichnet, dass**
die Signalverarbeitungs-Schaltung (40) ausgeführt ist zum:
Feststellen, dass der Zustand der Zielperson (500) ein Zustand von Bewusstseinsstörung ist, in Reaktion auf die Feststellung dahingehend, dass das biologische Signal nicht innerhalb des Standardbereiches liegt, sowie die Feststellung dahingehend, dass der Betrag von Veränderung des zerebralen Blutflusses unter dem Standardwert liegt, wobei die Zielperson (500) während des Zustandes von Bewusstseinsstörung bewusstlos ist; und
Feststellen, dass der Zustand der Zielperson (500) ein Schlafzustand ist, in Reaktion auf die Feststellung dahingehend, dass das biologische Signal nicht innerhalb des Standardbereiches liegt, sowie die Feststellung dahingehend, dass der Betrag von Veränderung des zerebralen Blutflusses nicht unter dem Standardwert liegt.

2. Biometrische Vorrichtung nach Anspruch 1, wobei der erste Detektor (20) des Weiteren so ausgeführt ist, dass er das biologische Signal registriert und ausgibt.

3. Biometrische Vorrichtung nach Anspruch 1, die des Weiteren umfasst:
einen zweiten Detektor (20, 50), der so ausgeführt ist, dass er das biologische Signal registriert und ausgibt.

4. Biometrische Vorrichtung nach einem der Ansprüche 1 bis 3, die des Weiteren umfasst:
eine Lichtquelle (10), die so ausgeführt ist, dass sie einen Lichtimpuls ausgibt, der einen Kopf der Zielperson (500) bestrahlt;
sowie
eine Steuerungs-Schaltung (30), wobei
der erste Detektor (20) ausgeführt ist zum:
Registrieren wenigstens eines Teils eines von dem Kopf zurückkehrenden reflektierten Impulses,
und
die Steuerungs-Schaltung (30) ausgeführt ist zum:
Veranlassen, dass die Lichtquelle (10) den Lichtimpuls emittiert, sowie
Veranlassen, dass der erste Detektor (20) eine interne Streuungs-Komponente, die im Inneren eines Gehirns der Zielperson (500) gestreut wird, aus dem reflektierten Lichtimpuls als das Gehirntätigkeits-Signal registriert und ausgibt.

5. Biometrische Vorrichtung nach einem der Ansprüche 1 bis 4, wobei das biologische Signal wenigstens einen Typ von Information enthält, die aus der Gruppe ausgewählt wird, die aus Puls, Perspiration, Respiration und Körpertemperatur besteht.

6. Biometrische Vorrichtung nach einem der Ansprüche 1 bis 5, wobei die Signalverarbeitungs-Schaltung (40) so ausgeführt ist, dass sie das Gehirntätigkeits-Signal nach Erfassen des biologischen Signals erfasst.

7. Biometrische Vorrichtung nach einem der Ansprüche 1 bis 6, wobei die Signalverarbeitungs-Schaltung (40) so ausgeführt ist, dass sie auf Basis des biologischen Signals und des Gehirntätigkeits-Signals feststellt und ausgibt, ob ein Puls der Zielperson (500) tachykardisch oder bradykardisch ist.

8. Biometrische Vorrichtung nach einem der Ansprüche 1 bis 7, wobei das biologische Signal Informationen über einen Blutfluss an einer Hautoberfläche der Zielperson (500) enthält.

9. Biometrische Vorrichtung nach Anspruch 4, wobei die Steuerungs-Schaltung (30) so ausgeführt ist, dass sie den ersten Detektor (20) veranlasst, des Weiteren aus dem reflektierten Lichtimpuls eine Oberflächenreflexions-Komponente, die an einer Hautoberfläche der Zielperson (500) reflektiert wird, zu registrieren,
und
ein Signal, das eine Veränderung der Oberflächenreflexions-Komponente anzeigt, als das biologische Signal auszugeben.

10. Biometrische Vorrichtung nach Anspruch 9, wobei die Steuerung-Schaltung (30) so ausgeführt ist, dass sie den ersten Detektor (20) veranlasst, die interne Streuungs-Komponente zu registrieren, indem er einen Teil des reflektierten Lichtimpulses nach Beginn von Abnahme einer Intensität des reflektierten Lichtimpulses registriert, und die Oberflächenreflexions-Komponente zu registrieren, indem er einen Teil des reflektierten Lichtimpulses vor Beginn von Abnahme der Intensität des reflektierten Lichtimpulses registriert.

11. Biometrische Vorrichtung nach Anspruch 4, wobei eine Zeit von einem Beginn von Bestrahlung mit dem Lichtimpuls bis zu einer Erfassung des Gehirntätigkeits-Signals länger ist als eine Zeit von dem Beginn von Bestrahlung mit dem Lichtimpuls bis zu einer Erfassung des biologischen Signals.

12. Biometrische Vorrichtung nach einem der Ansprüche 1 bis 11, wobei die Signalverarbeitungs-Schaltung (40) ausgeführt ist zum:
Treffen einer ersten Feststellung in Bezug auf den Zustand der Zielperson (500) auf Basis des biologischen Signals und Ausgeben eines ersten Signals, das ein Ergebnis der ersten Feststellung anzeigt, sowie
Treffen einer zweiten Feststellung in Bezug auf den Zustand der Zielperson (500) auf Basis des Gehirntätigkeits-Signals nach Ausgeben des ersten Signals, sowie Ausgeben eines zweiten Signals, das ein Ergebnis der zweiten Feststellung anzeigt.

13. Biometrische Vorrichtung nach Anspruch 12, die des Weiteren umfasst:
eine Kommunikations-Schaltung (60), die so ausgeführt ist, dass sie mit einer externen Vorrichtung (200) außerhalb der biometrischen Vorrichtung kommuniziert, wobei
wenn die Signalverarbeitungs-Schaltung (40) auf Basis wenigstens eines Signals, das aus der Gruppe ausgewählt wird, die aus dem biologischen Signal und dem Gehirntätigkeits-Signal besteht, feststellt, dass sich die Zielperson (500) nicht in einem normalen Zustand befindet, die Kommunikations-Schaltung (60) die externe Vorrichtung (200) darüber benachrichtigt, dass sich die Zielperson (500) nicht in dem normalen Zustand befindet.

## Revendications

1. Appareil biométrique comprenant :
un premier détecteur (20) conçu pour détecter et délivrer un signal d'activité cérébrale indiquant un état d'activité cérébrale chez une personne cible (500) ; et
un circuit de traitement de signal (40), le circuit de traitement de signal (40) étant conçu pour :
- acquérir le signal d'activité cérébrale,
- acquérir un signal biologique de la personne cible (500) qui est différent du signal d'activité cérébrale,
- déterminer, sur la base du signal biologique et du signal d'activité cérébrale un état de la personne cible (500), et
- générer et délivrer un signal indiquant l'état de la personne cible (500) ;
le signal d'activité cérébrale incluant des informations concernant un changement dans une quantité de flux sanguin cérébral chez la personne cible (500) ; et
le circuit de traitement de signal (40) étant conçu pour :
- déterminer, sur la base du signal biologique, si le signal biologique se situe à l'intérieur d'une plage standard ;
- déterminer, sur la base du signal d'activité cérébrale, si une quantité de changement dans le flux sanguin cérébral est inférieure à une valeur standard ; et
- en réponse à la détermination que le signal biologique se situe à l'intérieur de la plage standard, déterminer que l'état de la personne cible (500) est un état normal ;
**caractérisé en ce que** : le circuit de traitement de signal (40) est conçu pour :
- en réponse à la détermination que le signal biologique ne se situe pas à l'intérieur de la plage standard et de la détermination que la quantité de changement dans le flux sanguin cérébral est inférieure à la valeur standard, déterminer que l'état de la personne cible (500) est un état de conscience perturbée, la personne cible (500) étant inconsciente durant l'état de conscience perturbée ; et
- en réponse à la détermination que le signal biologique ne se situe pas à l'intérieur de la plage standard et à la détermination que la quantité de changement dans le flux sanguin cérébral n'est pas inférieure à la valeur standard, déterminer que l'état de la personne cible (500) est un état de sommeil.

2. Appareil biométrique selon la revendication 1, le premier détecteur (20) étant en outre conçu pour détecter et délivrer le signal biologique.

3. Appareil biométrique selon la revendication 1, comprenant en outre : un second détecteur (20, 50) conçu pour détecter et délivrer le signal biologique.

4. Appareil biométrique selon l'une quelconque des revendications 1 à 3, comprenant en outre :
une source de lumière (10) conçue pour émettre une impulsion de lumière irradiant une tête de la personne cible (500) ; et
un circuit de commande (30), le premier détecteur (20) étant conçu pour détecter au moins une partie d'une impulsion reflétée revenant de la tête, et le circuit de commande (30) étant conçu pour :
amener la source de lumière (10) à émettre l'impulsion de lumière, et
amener le premier détecteur (20) à détecter et délivrer une composante de diffusion interne diffusée à l'intérieur du cerveau de la personne cible (500) depuis l'impulsion de lumière reflétée comme signal d'activité cérébrale.

5. Appareil biométrique selon l'une quelconque des revendications 1 à 4, le signal biologique incluant au moins un type d'informations sélectionnées dans le groupe constitué d'une impulsion, d'une transpiration, d'une respiration, et d'une température corporelle.

6. Appareil biométrique selon l'une quelconque des revendications 1 à 5, le circuit de traitement de signal (40) étant conçu pour acquérir le signal d'activité cérébrale après l'acquisition du signal biologique.

7. Appareil biométrique selon l'une quelconque des revendications 1 à 6, le circuit de traitement de signal (40) étant conçu pour déterminer et émettre le fait qu'une impulsion de la personne cible (500) est une bradycardie ou une tachycardie, sur la base du signal biologique et du signal d'activité cérébrale.

8. Appareil biométrique selon l'une quelconque des revendications 1 à 7, le signal biologique incluant des informations sur un flux sanguin au niveau d'une surface cutanée de la personne cible (500).

9. Appareil biométrique selon la revendication 4,
le circuit de commande (30) étant conçu pour amener le premier détecteur (20) à détecter en outre une composante de réflexion de surface reflétée à une surface cutanée de la personne cible (500) depuis l'impulsion de lumière reflétée, et
délivrer un signal indiquant une variation dans la composante de réflexion de surface comme signal biologique.

10. Appareil biométrique selon la revendication 9,
le circuit de commande (30) étant conçu pour amener le premier détecteur (20) à détecter la composante de diffusion interne en détectant une portion de l'impulsion de lumière reflétée après qu'une intensité de l'impulsion de lumière reflétée a commencé à baisser, et
détecter la composante de réflexion de surface en détectant une portion de l'impulsion de lumière reflétée avant que l'intensité de l'impulsion de lumière reflétée commence à baisser.

11. Appareil biométrique selon la revendication 4, un moment depuis un début d'irradiation avec l'impulsion de lumière jusqu'à ce qu'une acquisition du signal d'activité cérébrale étant plus long qu'un moment à partir du début de l'irradiation avec l'impulsion de lumière jusqu'à une acquisition de signal biologique.

12. Appareil biométrique selon l'une quelconque des revendications 1 à 11, le circuit de traitement de signal (40) étant conçu pour :
effectuer une première détermination liée à l'état de la personne cible (500) sur la base du signal biologique, et délivrer un premier signal indiquant un résultat de la première détermination, et
après avoir délivré le premier signal, effectuer une seconde détermination liée à l'état de la personne cible (500) sur la base du signal d'activité cérébrale, et délivrer un second signal indiquant un résultat de la seconde détermination.

13. Appareil biométrique selon la revendication 12, comprenant en outre :
un circuit de communication (60) conçu pour communiquer avec un appareil externe (200) qui est externe à l'appareil biométrique,
lorsque le circuit de traitement de signal (40) détermine que la personne cible (500) ne se trouve pas dans un état normal sur la base d'au moins l'un sélectionné dans le groupe constitué du signal biologique et du signal d'activité cérébrale, le circuit de communication (60) notifiant l'appareil externe (200) que la personne cible (500) ne se situe pas dans l'état normal.
